(19) Europäisches Patentamt
European Patent Office
Office européen des brevets

(11) EP 2 864 767 B1

(12) **EUROPEAN PATENT SPECIFICATION**

(45) Date of publication and mention of the grant of the patent:
**23.11.2022 Bulletin 2022/47**

(21) Application number: **13731475.3**

(22) Date of filing: **21.06.2013**

(51) International Patent Classification (IPC):
***G01N 24/10*** *(2006.01)* ***G01R 33/60*** *(2006.01)*
***C07D 401/04*** *(2006.01)* ***C07K 14/78*** *(2006.01)*
***C07D 207/46*** *(2006.01)*

(52) Cooperative Patent Classification (CPC):
**C07D 401/04; C07D 207/46; G01N 24/10; G01R 33/60**

(86) International application number:
**PCT/GB2013/051644**

(87) International publication number:
**WO 2013/190329 (27.12.2013 Gazette 2013/52)**

(54) **ANALYTICAL METHOD ON THE BASIS OF ELECTRON SPIN RESONANCE (ESR) AND PROBE MOLECULE**

ANALYTISCHES VERFAHREN AUF BASIS VON ELEKTRONENSPINRESONANZ (ESR) UND SONDENMOLEKÜL

PROCÉDÉ D’ANALYSE BASÉ SUR LA RÉSONANCE DE SPIN ÉLECTRONIQUE (RSE) ET MOLÉCULE DE SONDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.06.2012 GB 201211159**

(43) Date of publication of application:
**29.04.2015 Bulletin 2015/18**

(73) Proprietor: **Aquaffirm Limited**
**Richmond TW10 6QA (GB)**

(72) Inventors:
- **AEPPLI, Gabriel**
  **London W1T 4TP (GB)**
- **KAY, Christopher W. M.**
  **London W1T 4TP (GB)**
- **SANCHANIA, Vishal A.**
  **Croydon CR0 4US (GB)**
- **BAKER, James R**
  **Lindfield, West Sussex RH16 2DE (GB)**
- **SCHUMACHER, Felix F.**
  **London W2 4NY (GB)**
- **CHESTER, Kerry A.**
  **London NW5 1HZ (GB)**
- **HEUTZ, Sandrine**
  **London W1T 4TP (GB)**

(74) Representative: **Haseltine Lake Kempner LLP**
**One Portwall Square**
**Portwall Lane**
**Bristol BS1 6BH (GB)**

(56) References cited:
- **FELIX F. SCHUMACHER ET AL: "In Situ Maleimide Bridging of Disulfides and a New Approach to Protein PEGylation", BIOCONJUGATE CHEMISTRY, vol. 22, no. 2, 16 February 2011 (2011-02-16), pages 132-136, XP055064120, ISSN: 1043-1802, DOI: 10.1021/bc1004685**
- **FELIX SCHUMACHER ET AL: "Modification of antibody disulfide bonds with maleimides", PROCEEDINGS OF THE ANNUAL MEETING, GERMAN SOCIETY FORBIOCHEMISTRY AND MOLECULAR BIOLOGY, XX, XX, 25 September 2011 (2011-09-25), pages 1-2, XP002697764,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SCHALLREUTER K U ET AL: "The role of calcium in the regulation of free radical reduction by thioredoxin reductase at the surface of the skin", JOURNAL OF INORGANIC BIOCHEMISTRY, ELSEVIER INC, US, vol. 28, no. 2-3, 11 October 1986 (1986-10-11), pages 227-238, XP026614511, ISSN: 0162-0134, DOI: 10.1016/0162-0134(86)80086-1 [retrieved on 1986-10-11]**
- **KÁLMÁN HIDEG ET AL: "Recent results in chemistry and biology of nitroxides", JOURNAL OF HETEROCYCLIC CHEMISTRY, vol. 42, no. 3, 1 April 2005 (2005-04-01), pages 437-450, XP055080548, ISSN: 0022-152X, DOI: 10.1002/jhet.5570420311**
- **CHRIS P. RYAN ET AL: "Tunable reagents for multi-functional bioconjugation: reversible or permanent chemical modification of proteins and peptides by control of maleimide hydrolysis", CHEMICAL COMMUNICATIONS, vol. 47, no. 19, 1 January 2011 (2011-01-01), page 5452, XP055062850, ISSN: 1359-7345, DOI: 10.1039/c1cc11114k**


Remarks:

The complete document including Reference Table(s) and the Sequence Listing(s) can be downloaded from the EPO website

## Description

The present invention relates to biosensor technology using electron spin resonance (ESR).

There is an ever increasing need for biosensor technologies to solve problems posed by modern medicine. Biosensors provide a method for detecting specific analytes of interest in a single and, sometimes, a range of matrixes. For example, the analyte of interest may be a toxic compound found in contaminated water sources, a biomolecule whose levels are raised or diminished during disease, or a candidate drug molecule interacting with a target. Currently, there are many techniques which employ optical1, electrochemical/redox[2], piezoelectric[3], surface plasmon resonance[4] and other physical phenomena to sense the presence of particular analytes. When truly quantitative information is sought, as is increasingly the case for modern biomedicine, most of them entail relatively sophisticated metrology and chemistry (e.g. careful preparation of surfaces) for the sensing of a particular analyte. Furthermore, personalised medical care demands rapid, point-of-care, multiplexed, matrix insensitive, in-situ/implantable biosensor technologies.

Electron spin resonance, otherwise termed electron paramagnetic resonance (EPR), has previously been used as a platform for biosensing. One such EPR biosensor is the free radical assay technique (FRAT)[14, 15]. FRAT makes use of free radical labelled analogues of small antigens whose spectra differ in the absence and presence of their specific antibodies. The difference is due to changes in the tumbling motion of the antigens upon complex formation with their specific antibodies. When the bound labelled antigen-analogue antibody complex has formed, it is isolated and placed into a sample containing an unknown amount of unlabelled naturally occurring antigen i.e. a test sample. The antigen within the test sample displaces the analogous labelled antigen, resulting in a free labelled antigen EPR spectrum. This displacement is highly dependent on the labelled antigen having a lower affinity to the antibody in comparison to the native antigen. The level of displacement can be compared to a standard curve to estimate the concentration of a particular antigen in a test solution. U.S. Pat. No. 3,690,834 describes the range of labelled antigen and method in more detail. Nitroxide spin labelled antigens have also been used to map the depth of antibody-antigen binding sites[17]. This method has the disadvantage that: every antigen needs to be labelled and every antigen is different, labelled antigens need to have a lower affinity for the antibody than the native antigen and the antibody-antigen complex must be isolated prior to its exposure into the test sample.

A membrane immunoassay, namely, SMIA (spin membrane immunoassay) uses a signal amplification technique in the form of complement mediated liposome lysis to sense the presence of specific antibodies[18]. Briefly, liposomes activated with antigen by covalently binding them to the polar headgroups of the liposomes making them accessible for potential binding antibodies. The liposome itself is composed of an array of lipid bilayers which encapsulate N,N-dimethyl-N-(2,2,6,6-tetramethyl-4-piperidinyl-1-oxy-l2)-hydroxylethylammonium chloride (TEMPOcholine chloride). The latter molecules are paramagnetic and have a strongly dipolar broadened spectrum when packed into the liposomes. Upon complement lysis (antibody-antigen complex formation) the TEMPOcholine is released, resulting in a highly isotropic TEMPOcholine EPR spectrum. The level of lysis is correlated to the levels of the complex formation. This method has the disadvantages of surface biosensors; antigen must be immobilized on liposome surfaces.

It would be desirable to provide alternative methods, ideally improved methods, for detecting species of interest.

## Summary of the Invention

In a first aspect, there is provided an electron spin resonance (ESR) analytical method comprising:

> providing a probe molecule capable of binding selectively to a target, wherein a paramagnetic moiety is bound to the probe molecule via two thioether linkages, wherein each thioether linkage is a carbon-sulphur-carbon linkage and one carbon of the thioether linkage is part of the probe molecule, and the other carbon is part of either the paramagnetic moiety or a linker group that binds to the paramagnetic moiety;
> contacting the probe molecule with a first sample to form a mixed sample and obtaining an electron spin resonance signal of the mixed sample;

wherein the probe molecule is selected from an antibody fragment, an antibody and an aptamer. The first sample may or may not contain the target. Preferably, the electron spin resonance signal of the probe molecule when bound to the target is different from an electron spin resonance signal of the probe molecule that is not bound to the target.

The method of the present invention is defined in claim 1.

In a second aspect, there is provided a probe molecule capable of binding selectively to a target, wherein a paramagnetic moiety is bound to the probe molecule via two thioether linkages; wherein each thioether linkage is a carbon-sulphur-carbon linkage and one carbon of the thioether linkage is part of the probe molecule, and the other carbon is part of either the paramagnetic moiety or a linker group that binds to the paramagnetic moiety
wherein the probe molecule is selected from an antibody fragment, an antibody and an aptamer. Preferably, the electron spin resonance signal of the probe molecule when bound to the target is different from an electron spin resonance signal

of the probe molecule that is not bound to the target.

The probe molecule of the present invention is defined in claim 11.

Also described is an apparatus comprising an electron spin resonance spectrometer, the apparatus adapted to obtain an electron spin resonance signal of a mixed sample, the mixed sample formed by contacting a probe molecule capable of binding selectively to a target with a first sample that may or may not contain the target, wherein the probe molecule is selected from an antibody fragment, an antibody and an aptamer,

wherein a paramagnetic moiety is bound to the probe molecule via two thioether linkages wherein each thioether linkage is a carbon-sulphur-carbon linkage and one carbon of the thioether linkage is part of the probe molecule, and the other carbon is part of either the paramagnetic moiety or a linker group that binds to the paramagnetic moiety; wherein the apparatus can detect the presence and, optionally, the concentration of the target in the mixed sample; wherein the apparatus comprises the mixed sample. Preferably, the electron spin resonance signal of the probe molecule when bound to the target is different from an electron spin resonance signal of the probe molecule that is not bound to the target.

The apparatus is not part of the present invention.

Also described is a molecule of the formula (III)

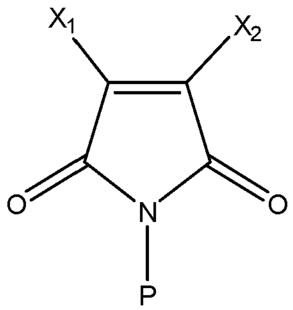

formula (III),

wherein X1 and X2 are each leaving groups and P is a paramagnetic moiety, wherein X1 and X2 are each independently selected from a halide, OR, SeR and SR, wherein R in any of OR, SR and SeR is a hydrocarbon group.

The present inventors have found that the techniques they have developed are generally sensitive and can allow targets to be detected at low concentrations in carrier mediums, e.g. whole blood, that are not readily analysed by optical techniques. An advantage of some embodiments is that the method is not a competition assay relying on displacement, but instead they measure directly the binding of antibody to antigen. Some embodiments of the invention do not use a surface and therefore avoid surfacerelated problems and other disadvantages encountered in the biosensing / biophysics field. Embodiments of the present invention have one or more of the following advantages:

i. No need for surface preparation and respective antigen compatibility as there is no surface directly involved in sensing.
ii. It is possible to use opaque carrier mediums for target species, such as blood. Here, optical technologies would most likely fail as a complex medium like blood contains other molecules that may interfere with their method of detection.
iii. The method can provide solution phase binding constants. Surface technologies are frequently used to estimate binding constants but are notorious for overestimating them.
iv. Working volumes can be minimized using ferroelectric inserts (semi-nanolitre volumes).
v. Sensitivity can be improved and/or working volumes can be reduced by using optical detection of electron paramagnetic resonance[16]; because this is a mixed microwave/optical measurement, disadvantages of purely optical or microwave techniques may be avoided.
vi. Potential for high throughput and automation.
vii. Potential for real time monitoring of binding events using a mixer system or specially designed sample holder.
viii. Can be temperature controlled and therefore be used for denaturation profiling or antibody-antigen interactions i.e. thermal stability shift assays for drug discovery.
ix. Unlike so many biosensors that require careful and a systematic range of steps in order to detect binding, the invention can adopt a simple mix and measure metrology.
x. EPR spectrometers are portable, thereby having point-of-care potential.

The present inventors have also found that they can obtain a continuous spectrum, which provides for increased sensitivity and increased information, particularly concerning the nature of the bound state. With a full spectrum potentially containing sharp features, it is also easier to discriminate against nonspecific binding events than for techniques providing only a single number such as surface plasmon resonance or photoluminescence/fluorescent yield.

As briefly mentioned above, relative to other (primarily purely optical) binding assays, the method has the advantages that it allows the skilled person to work with carrier mediums (such as whole blood - see below) with non-optimal optical properties and still obtain meaningful quantitative information. Embodiments of the method use microwave absorption, measured using a cavity resonance or potentially by optical means[16], which can be easily calibrated using reference molecules. The molecular tumbling that the present inventors have observed requires less sophisticated modelling than fluorescence and other optical methods, coupling to electronic excitations, to obtain specific site geometries.

An important aspect of the method described herein is that it can be completely solution based. There is no perturbation of the binding mechanics due to surface induced denaturation as incurred in common methods such as ELISA, providing near native detection. Hence, there is no need for surface regeneration or optimization as is required for the vast majority of competing technologies and allows for reuse of the sample holders after a simple wash step, making it relatively economical.

Furthermore, by having a highly targeted solution assay and not a surface assay, the present inventors eliminate non-specific adsorption problems. Any non-specific binding can be isolated to the biomolecules avidity or the buffer / matrix conditions, which means that non-specific binding will be less of an issue than for competing technologies, and indeed has not been a problem for the cancer marker for which this system has thus far been tested.

While antibodies themselves have been spin labelled before, to the inventors' knowledge, no one has used them successfully to detect the presence or concentration of another species. It has been shown that the EPR spectrum of 2,2,6,6-tetramethyl-4-aminopiperidine-I-oxyl labelled IgGM antibody on the n-acetylneuraminic acid residue within the Fab fragment of the antibody is sensitive to change in tumbling motions; as shown by temperature and viscosity experiments only[19]. 4-amino-2,2,6,6-tetramethylpiperidin-I-oxyl has been used to label carbohydrates in rabbit anti-DNP and used to monitor the binding of ε-Dnp-iysine. However, no change was visible in the presence and absence of ε-Dnp-lysine[20]. Accordingly, labelled antibody binding events with antigen, monitored by EPR would not have been obvious to those skilled in the art.

## Brief Description of the Figures

Figures 1(a) to 1(e) illustrate schematically an embodiment of a method of the Invention.

Figures 2(a) to 2(d) illustrate binding constant acquisition using an embodiment of the method of the invention.

Figures 3(a) to 3(t) show raw and simulated data acquired to generate Figures 2(a) to 2(d).

Figures 4(a) to (h) illustrate biophysical analysis of anti-CEA complex using thermal denaturation - EPR Spectra.

Figures 5(a) and 5(b) illustrate biophysical analysis of anti-CEA complex using thermal denaturation - denaturation profile of EPR spectra in Figure 4.

Figures 6(a) and (b) illustrate anti-CEA binding kinetics followed by EPR.

## Detailed Description

The present invention provides the first to fourth aspects mentioned above. Optional and preferred features of the aspects will now be described. Unless otherwise stated, any optional or preferred feature may be combined with any of the aspects or any of the other optional or preferred features.

As described, the paramagnetic moiety is bound to the probe molecule via two thioether linkages. A thioether linkage is a carbon-sulphur-carbon linkage. One carbon of the thioether linkage is part of the probe molecule, and the other carbon is part of a paramagnetic moiety or linker group or species, which may be as described herein, that binds to the paramagnetic moiety. In an embodiment, the paramagnetic moiety may be bound to the probe molecule by more than two thioether linkages.

In an embodiment, the electron spin resonance signal of the mixed sample is compared with an electron spin resonance signal of the probe molecule that is not bound to the target, with a difference in the signal indicating the

presence of the target in the mixed sample. In an embodiment, the electron spin resonance signal of the mixed sample is compared with an electron spin resonance signal of the probe molecule that is not bound to the target that is present in a reference sample. In an embodiment, the reference sample is substantially the same as, or the same as, the first sample, except lacking the target, with a difference in the signal indicating the presence of the target in the mixed sample. "Substantially the same" indicates that any differences between the samples do not have an effect on the electron spin resonance signal. Target may be termed an antigen herein.

The method may involve detecting a difference between the electron spin resonance signal of the mixed sample and the electron spin resonance signal of the probe molecule that is not bound to the target, which may be in a reference sample as described herein. The apparatus may be adapted to detect a difference between the electron spin resonance signal of the mixed sample and the electron spin resonance signal of the probe molecule that is not bound to the target, which may be in a reference sample as described herein.

In an embodiment, the mixed sample contains the target, and the concentration of the target in the mixed sample and/or the first sample is determined from the electron spin resonance signal of the mixed sample. The apparatus may be adapted to calculate the concentration of the target in the mixed sample and/or the first sample from the electron spin resonance signal of the mixed sample.

In an embodiment, the concentration is determined by using a predetermined relationship between the electron spin resonance signal and the concentration of the target in a sample. In an embodiment, the method involves a calibration step to determine the relationship between the electron spin resonance signal and the concentration of the target in a sample. The calibration step may involve determining the electron spin resonance signal of a plurality of reference samples, each reference sample containing the target at a different concentration from the other reference sample(s). The reference samples may each be substantially the same as, or the same as, the first sample, except they each may contain a different concentration of target compared to the first sample. In an embodiment, the apparatus is adapted to carrying out a calibration step as described herein.

Optionally, a standard addition is carried out on the mixed sample to determine the concentration of the target in the mixed sample. This is particularly effective where the mixed sample contains a biological fluid. Standard addition is sometimes termed "spiking". In standard addition, a sample containing a known amount of target (sometimes termed analyte) is added to the mixed sample, in which the concentration of target is unknown, and the electron spin resonance signal measured; this is repeated for different amount of target added to the sample. The variance of the electron spin resonance with the addition of the known amount of target added to the mixed sample can be used to extrapolate the concentration of the target in the mixed sample. Standard addition or spiking is known to the skilled person.

In an embodiment, the electron spin resonance signal of the mixed sample are compared with the electron spin resonance signal of a first reference sample that contains the probe molecule and the target and the electron spin resonance signal of a second reference sample that contains the probe molecule, but lacks the target. Optionally, the mixed sample, first reference sample and second reference sample all contain a biological fluid, which may be as described below. Optionally, a calibration is carried out, wherein the target is added to a reference sample, which contains the probe molecule and which may contain a biological fluid, at a plurality of known concentrations, and recording the electron spin resonance signal, and a relationship between the electron spin resonance signal and the concentration of target in the reference sample. The relationship may then be used to calculate the concentration of the target in the mixed sample (and/or first sample) from the electron spin resonance signal of the mixed sample.

In an embodiment, the method comprises the calibration step to determine a relationship between the electron spin resonance signal and the concentration of target in a sample, and the relationship is used to determine, from the ESR signal of the mixed sample, the concentration of the target in the mixed sample and/or the first sample.

In an embodiment, the concentration is determined by using a predetermined relationship between the mean square deviation of electron spin resonance signal (the deviation being from the probe molecule that is not bound to the target, which may be in a reference sample lacking the target, as described herein) and the concentration of the target in a reference sample containing the target. The reference samples lacking and containing the target should provide suitable samples for calibration of the concentration of the target in the first sample. The reference samples lacking and containing the target may be substantially the same as or the same as the first sample, except for the concentration of the target.

In an embodiment, the method comprises a calibration step to determine a relationship between the mean square deviation of electron spin resonance signal (the deviation being from the probe molecule that is not bound to the target, which may be in a reference sample lacking the target, as described herein) and the concentration of the target in a reference sample, and the relationship is used to determine, from the ESR signal of the mixed sample, the concentration of the target in the mixed sample and/or first sample.

Optionally, the method involves taking the mean squared deviation of the target EPR spectrum at a concentration or at a plurality of concentrations; the EPR spectrum or spectra may be normalised to their maximum and the relevant transitions aligned (e.g. in the case of nitroxide, the -1 0 +1 (N = 1) transition) before computing their mean squared deviations, optionally as described in equation 1.

$$\frac{1}{N}\sum(Xc - Xo)^2 \qquad \text{Eq. (1)}$$

wherein N is the number of data points in the EPR spectrum, *Xc* is the spectrum containing the target (or antigen) at concentration c where c→∞, Xo is the spectrum containing no target (or antigen) i.e. c-70. A single titre may represent a single data point towards a binding curve, from which thermodynamic binding constants can be extracted. In aligning transitions, for a first derivative EPR spectrum, the point of alignment would be the point in the spectrum at the relevant transition where the signal line crosses the x axis, or for an absorbance spectrum, this would be the peak of absorbance. In the calibration step, the concentration of the target may be known and the mean square deviation calculated for a plurality of concentrations of target species, and the mean square deviation for each target concentration used to determine a relationship between the concentration of target and the mean square deviation. The mean square deviation of the mixed sample (in which the concentration of the target may not be known) can be obtained, and using the relationship between the mean square deviation and concentration of the target, the concentration of the target in the mixed sample (and/or the first sample) can be determined.

Optionally, the method involves fitting electron spin resonance data using the following equation 2:

$$fB = \frac{([L]+[Bi]+Kd)-\sqrt{(([L]+[Bi]+Kd)^2-4\,.[L].[Bi])}}{2\,.[Bi]} \qquad \text{Eq. (2)}$$

where *fB* is the fraction of the target bound to the probe molecule, [*L*] is the target molecule concentration, [*Bi*] is the initial probe molecule concentration, and *Kd* is the dissociation constant.

Optionally, the method involves, optionally in a calibration step as described herein, calculating thermodynamic data from the electron spin resonance signal, including, but not limited to, the binding constant of the probe molecule and target.

The concentration of probe molecule is preferably the same in all samples compared, and all conditions for the measurement of the electron spin resonance signals are the same (e.g. conditions including, but not limited to, temperature, pressure, and settings of the ESR spectrometer). The concentration probe molecule can be selected to ensure a sufficiently strong EPR signal is seen for a given concentration of target in a sample.

In an embodiment, the probe molecule capable of binding selectively to a target, in the first sample, mixed sample and/or the reference sample(s) is provided in a carrier medium, and the probe molecule may be added to or dissolved in the carrier medium. The carrier medium is preferably in liquid form. The carrier medium may be or comprise any liquid in which the target can be suspended or dissolved. In an embodiment, the carrier medium comprises water. In an embodiment, the carrier medium comprises a biological fluid. A biological fluid may be a fluid that has been obtained from a subject, which may be a human or an animal. In an embodiment, the carrier liquid comprises an undiluted biological fluid. An undiluted biological fluid in the present context is a biological fluid obtained from a subject, e.g. a human or animal, that has not been diluted with another liquid, although additives such as a buffer and/or an anticoagulant, may be present in the undiluted biological fluid. The biological fluid may be selected from blood, urine, tears, saliva, sweat, cerebrospinal fluid, cell lysate. The biological fluid may be selected from whole blood and blood plasma. In an embodiment, the target may be a food substance.

The carrier medium may further comprise a buffer and/or saline. The buffer may comprise a phosphate, e.g. an alkali metal phosphate and/or an alkali metal hydrogen phosphate, e.g. selected from, $Na_2HPO_4$ and $KH_2PO_4$. The saline may comprise an alkali metal chloride, e.g. selected from NaCl and KCl.

The probe molecule may be present in the carrier medium in any of samples (e.g. the first sample, reference sample and/or the mixed sample) in a concentration of at least 10 nM, optionally at least 20 nM, optionally at least 50 nM, optionally at least 100 nM. The probe molecule may be present in the carrier medium in any of samples (e.g. the first sample, reference sample and/or the mixed sample) in a concentration of 100 μM or less, optionally 50 μM or less, optionally 20 μM or less, optionally 10 μM or less.

In contacting the probe molecule with a first sample, the probe molecule and first sample are preferably allowed to reach equilibrium in the mixed sample before the electron spin resonance signal of the mixed sample is obtained. Equilibrium may be achieved by allowing the mixed sample to stand for an appropriate period of time. The period of time can be determined by the skilled person.

In an embodiment, either the probe molecule or the target is disposed on, and optionally bound to, a support, optionally a solid support, with the other of the probe molecule and the target optionally being in a carrier medium, which may be as described herein. The solid support may comprise a plastic substrate, which may be in the form of beads or a flat surface. Optionally the support is a living cell. Optionally, the target is bound to the support, and optionally the amount of target on the support may be known or unknown; the probe molecules may be added to the support, with the probe molecules preferably in a carrier medium, which may be as described herein; the electron spin resonance signal

may be obtained. The present inventors have found that using a support can result in signal amplification.

The paramagnetic moiety is bound to the probe molecule via two thioether linkages. In an embodiment, the probe molecule comprises at least one cysteine residue, and the paramagnetic moiety is bound to the probe molecule via a thioether linkage formed by the sulphur atom of the at least one cysteine residue. In an embodiment, the probe molecule comprises two cysteine residues, and the paramagnetic moiety is bound to the probe molecule via two thioether linkages formed by the sulphur atoms of the cysteine residues.

In an embodiment, the paramagnetic moiety is bound to the probe molecule in a moiety of formula (I)

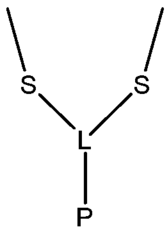

formula (I),

wherein each S forms part of a thioether linkage, such that the sulphur atom is bound to the probe molecule via a carbon atom, L is a linker moiety and P is the paramagnetic moiety. Optionally each S is the sulphur atom of a cysteine residue.

Optionally, L is or comprises a 5- or 6-membered ring. The 5- or 6-membered ring may be a hydrocarbon 5- or 6-membered ring or a heterocyclic 5- or 6-membered ring. Optionally L is or comprises a maleimide group. Optionally L is or comprises a heteroaryl group. The term "heteroaryl" as used herein may mean a monocyclic or bicyclic radical of 5 to 12 ring atoms having at least one aromatic ring containing four to eight atoms per ring, incorporating one or more N, O, or S heteroatoms, the remaining ring atoms being carbon, with the understanding that the attachment point of said heteroaryl radical will be on said aromatic ring. As well known to those skilled in the art, heteroaryl rings have less aromatic character than their all-carbon counter parts. Thus, for the purposes of the invention, a heteroaryl group need only have some degree of aromatic character.

Examples of heteroaryl moieties include monocyclic aromatic heterocycles having 5 to 6 ring atoms and 1 to 3 heteroatoms including but not limited to, pyridinyl, pyrimidinyl, pyrazinyl, pyridazinone, pyrrolyl, pyrazolyl, imidazolyl, triazolinyl, thiophenyl and oxadiaxolinyl which can optionally be substituted with one or more, preferably one or two substituents selected from hydroxy, cyano, alkyl, alkoxy, thio, lower haloalkoxy, alkylthio, halo, haloalkyl, alkylsulfinyl, alkylsulfonyl, halogen, amino, alkylamino, dialkylamino, aminoalkyl, alkylaminoalkyl, dialkylaminoalkyl, nitro, alkoxycarbonyl and carbamoyl, alkylcarbamoyl and dialkylcarbamoyl and aryloxy such as benzyloxy

Optionally, the paramagnetic moiety is bound to the probe molecule via a maleimide group that binds to two sulphur atoms, each sulphur atom forming part of a thioether linkage, and optionally each sulphur atom of the thioether linkage is part of a cysteine residue of the probe molecule. Optionally, the paramagnetic moiety P is bound to the probe molecule in a moiety of formula (II)

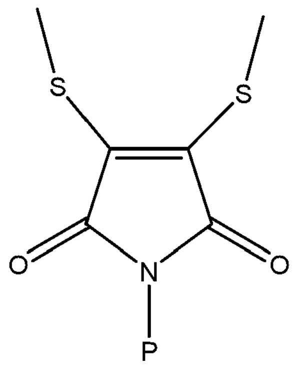

formula (II).

Optionally, each S forms part of a thioether linkage, such that the sulphur atom is bound to the probe molecule via a carbon atom, and P is the paramagnetic moiety. Optionally each S is the sulphur atom of a cysteine residue, and optionally each cysteine residue forms part of a variable or constant domain of an antibody or an antibody fragment.

Optionally, the paramagnetic moiety has been bound to a probe molecule having a disulphide bond by inserting the paramagnetic moiety between the sulphur atoms of the disulphide bond, such that the paramagnetic moiety is bound to the probe molecule via two thioether linkages, optionally via a linker group.

The probe molecule is selected from an antibody, an antibody fragment, and an aptamer. Optionally, the

antibody fragment is or is derived from a single chain variable fragment (scFv). Optionally, the probe molecule is or is derived from a cystine stabilised antibody, a cystine stabilised antibody fragment, or a cystine stabilised aptamer. Cystine stabilised antibodies or antibody fragments are sometimes termed disulphide stabilised antibodies or antibody fragments. Optionally, the probe molecule is or is derived from a cystine stabilised single chain variable fragment. A cystine stabilised species is a species containing cystine. Cystine is formed from two cysteine amino acids, which may form part of a peptide chain in the probe molecule, wherein the two sulphur atoms of the cysteines have been oxidised and covalently bonded to one another to form a disulphide bond. Preferably, the probe molecule in any one of the aspects of the invention is derived from a probe molecule having a cystine (e.g. a cystine stabilised single chain variable fragment), wherein the disulphide bond of the cystine has been cleaved and each sulphur atom of each cysteine bonded to the paramagnetic moiety as part of a thioether linkage, optionally via a linker group, for example in a moiety of formula (I) or (II); preferably each cysteine is in a variable fragment of the probe molecule that can bind to the target. The cystine stabilised antibody or antibody fragment, e.g. single chain antibody fragment, may be produced by substitution, in an antibody or antibody fragment capable of binding selectively to the target, of cysteine for two non-cysteine amino acids; optionally at the any one of the following combination of positions: position 44 of the heavy chain variable region (VH 44) and position 100 of the light chain variable region (VL100), on the basis of Kabat numbering; VH105 and VL43; VH108 and VL55; VH106 and VL56; VH101 and VL46; VH101and VL55. Some disulphide stabilised Fv fragments are described, for example, in Nat Biotechnol. 1996 Oct;14(10):1239-45, an article authored by Reiter et al.

If the probe molecule comprises a human or humanised antibody or an antibody fragment, the antibody or the antibody fragment may be selected from one or more of the classes IgA, IgD, IgE, IgG and IgM. The antibody or antibody fragment may be derived from a mammal, including, but not limited to, a mammal selected from a human, a mouse, a rat, a rabbit, a goat, a sheep, a horse, llama, camel and shark antibodies.

If the probe molecules comprise an aptamer, the aptamer may be selected from a peptide aptamer, a DNA aptamer and a RNA aptamer.

In an embodiment, the probe molecule is or comprises an antibody or antibody fragment, having a variable domain, which may be a light chain variable domain or heavy chain variable domain, which can bind selectively to the target, and both of the S atoms in formula (I) are attached to the variable domain as part of a thioether linkage. In an embodiment, the probe molecule is or comprises an antibody or antibody fragment, having a heavy chain variable domain and a light chain variable domain, which together can bind selectively to the target, and one of the S atoms in formula (I) is attached to the heavy chain variable fragment and the other of the S atoms in formula (I) is attached to the light chain variable fragment. In an embodiment, the probe molecule is or comprises a single chain variable fragment of an antibody, the single chain variable fragment comprises a heavy chain and a light chain, which together can bind selectively to the target, and one of the S atoms in formula (I) is attached to the heavy chain and the other of the S atoms in formula (I) is attached to the light chain; optionally, a linker sequence links the heavy chain and the light chain, the linker sequence optionally containing from 10 to 25 amino acid residues, optionally 12 to 16 amino acid residues, optionally 14 to 16 amino acid residues, the amino acid residues optionally selected from glycine, serine and threonine, and optionally the linker sequence is selected from $(GLy_4Ser)_3$ (SEQ ID NO: 2) and $(Gly_4Ser)_4$ (SEQ ID NO: 3).

In an embodiment, the single chain variable fragment is or comprises a Seq. ID No. 1,

**1**QVKLEQSGAEVVKPGASVKLS**C**KASGFNIKDSYMHWLRQGPGQ**C**LEWIGWIDPENGDTEYAPKFQ

**66** GKATFTTDTSANTAYLGLSSLRPEDTAVYY**C**NEGTPTGPYYFDYWGQGTLVTVSSGGGGSGGGGSG

**131** GGGSGGGGSENVLTQSPSSMSASVGDRVTIA**C**SASSSVPYMHWFQQKPGKSPKLLIYSTSNLASGVP

**197** SRFSGSGSGTDYSLTISSVQPEDAATYY**C**QQRSSYPLTFG**C**GTKLEIK (SEQ. ID. NO. 1),

wherein the underlined cysteines C are each bound to the paramagnetic moiety, optionally in a moiety of formula (I) or (II); optionally each sulphur atom of the cysteines C forms part of a thioether linkage and, optionally, is one of the sulphur atoms in a moiety of formula (I) or (II).

The paramagnetic moiety may be any moiety containing a paramagnetic atom. The paramagnetic moiety may be selected from aromatic radicals, e.g. aromatic radical anions, including, but not limited to, trityl radicals, benzene, $C_8H_8$, napthalene, anthracene, pyrene, perylene, biphenylene.

The paramagnetic moiety may contain a paramagnetic metal atom, including, but not limited to, copper(II), vanadium, gallium, gadolinium(III), manganese, iron, chromium, molybdenum, titanium, and nickel. The paramagnetic metal atom may be coordinated to a ligand. In an embodiment, the paramagnetic metal atom is a transition metal atom having a $d^1$ electron structure or $d^9$ electron structure. Transition metal atoms having a $d^1$ electron structure include, but are not limited to, $V^{4+}$ (e.g. in $VO^{2+}$), $Cr^{5+}$ and $Ti^{3+}$. Transition metal atoms having a $d^9$ electron structure include, but are not limited to, $Cu^{2+}$ and Ni+ (both of which are S ½ systems).

The paramagnetic moiety may comprise an organometallic species, which may include, but is not limited to a metal phthalocyanine, a metal porphyrin, and an iron-sulphurcontaining species.

The paramagnetic moiety may comprise an organic species, e.g. an aryl or heteroaryl species, having a triplet state, which may include, but is not limited to, napthalene, quinoline, isoquinoline, quinoxaline, anthracene, phenanthrene and pyrene.

The paramagnetic moiety may comprise a spin trap molecule, which may be in paramagnetic or diamagnetic form. The spin trap molecule may be selected from, but is not limited to, 5,5-dimethyl-pyrroline N-oxide (DMPO) and 2,2-diphenyl-1-picrylhydrazyl (DPPH).

The paramagnetic moiety may comprise a species selected from a fullerene-containing species, e.g. N@C60, biradical and other pluralities of label e.g. dendrimeric species and their derivatives, and single-molecule magnets.

Optionally, the paramagnetic moiety comprises a nitroxide group. Optionally, the paramagnetic moiety comprises a nitroxide group, which forms part of a 5- or 6-membered ring.

Optionally, the paramagnetic moiety or P comprises or is of the formula (IV)

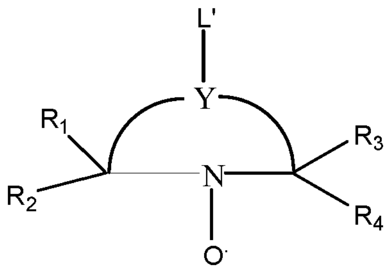

formula (IV)

Wherein $R_1$ to $R_4$ are each independently an optionally substituted hydrocarbon, optionally containing from 1 to 4 carbon atoms, excluding any substituents;

Y is an optionally substituted hydrocarbon bridging group containing from 2 to 4 carbon atoms, and optionally one or more heteroatoms,

and L' is a linker species binding to the probe molecule, which may be the same as or bound to the linker species L defined above. Optionally, all of $R_1$ to $R_4$ are methyl. Optionally, Y is a $C_2$ or $C_3$ alkylene group, in which one of the carbons is covalently bonded to L'. Optionally L' is a single bond or an alkylene group, for example an alkylene group having from 1 to 3 carbons.

In an embodiment, the paramagnetic moiety or P is of the formula (V)

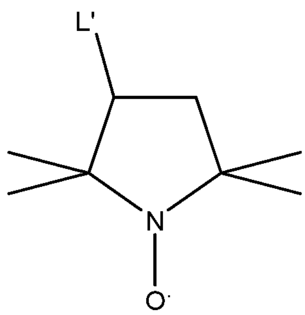

formula (V)

L' is a linker species binding to the probe molecule, which may be the same as or bound to the linker species L defined above.

In an embodiment, a paramagnetic moiety is bound to the probe molecule in a moiety of formula (VI)

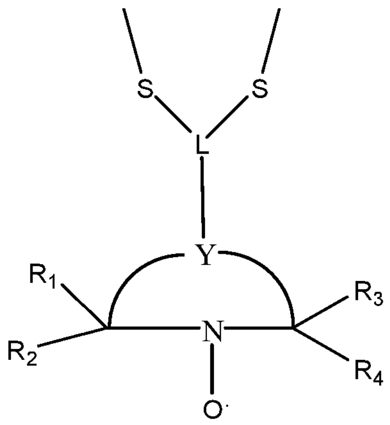

(formula VI),

wherein each S forms part of a thioether linkage that binds to the probe molecule, L is a linker moiety, R1 to R4 are each independently an optionally substituted hydrocarbon, optionally containing from 1 to 4 carbon atoms, excluding any substituents;

Y is an optionally substituted hydrocarbon bridging group containing from 2 to 4 carbon atoms, and optionally one or more heteroatoms. Optionally, all of R1 to R4 are methyl. Optionally each S is the sulphur atom of a cysteine residue.

In an embodiment, a paramagnetic moiety is bound to the probe molecule in a moiety of formula (VII)

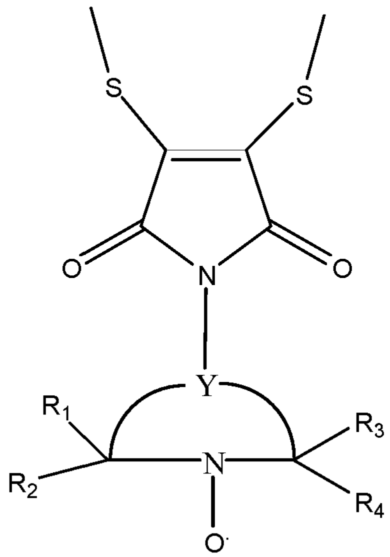

formula (VII),

wherein each S forms part of a thioether linkage that binds to the probe molecule, R1 to R4 are each independently an optionally substituted hydrocarbon, optionally containing from 1 to 4 carbon atoms, excluding any substituents; Y is an optionally substituted hydrocarbon bridging group containing from 2 to 4 carbon atoms, and optionally one or more heteroatoms. Optionally, all of R1 to R4 are methyl. Optionally each S is the sulphur atom of a cysteine residue. Optionally a linker species L' is present between N of the maleimide ring and Y, and L' may be as defined herein.

Optionally, in any of the formulae above, Y is selected from $C_{2-4}$ alkylene, wherein one of the carbons of the alkylene is directly bonded to L, L' or N, as appropriate; $C_{2-4}$ alkenylene, wherein one of the carbons of the alkenylene is directly bonded to L, L' or N, as appropriate; (CH-O-$CH_2$), wherein C is directly bonded to L, L' or N, as appropriate; and CH-S-$CH_2$), wherein C is directly bonded to L, L' or N, as appropriate.

In an embodiment, a paramagnetic moiety is bound to the probe molecule in a moiety of formula (VIII)

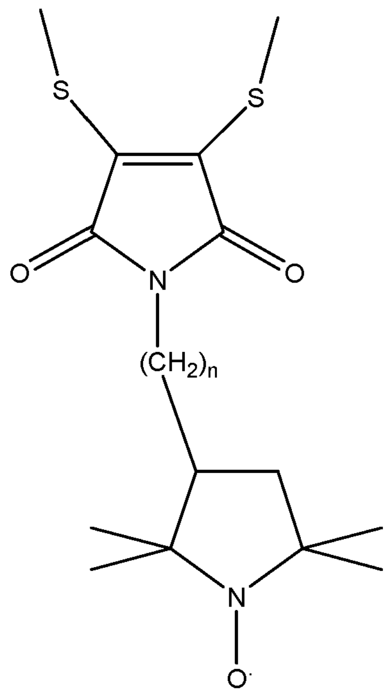

formula (VIII),

wherein each S forms part of a thioether linkage that binds to the probe molecule and n is from 0 to 4, optionally 1 to 4, optionally 0, 1, 2 or 3. Optionally each S is the sulphur atom of a cysteine residue.

The probe molecule is capable of binding selectively to a target. The target may be selected from, but is not limited to, proteins, glycopeptides, polypeptides, antibodies, nanoparticles, drugs, toxins, harmful gases, hazardous chemicals, explosives, viral particles, cells, multi-cellular organisms, cytokines and chemokines, ganietocyte, organelles, lipids, nucleic acid sequences, oligosaccharides, chemical intermediates of metabolic pathways and macromolecules.

If the target is or comprise a protein, the protein may be selected from, but is not limited to, native proteins, denatured proteins, protein fragments, and prokaryotically or eukaryotically expressed proteins. Protein may have its normal meaning in the art, and most preferably 'protein' refers to a polypeptide molecule. Such polypeptide may comprise modifications such as glycosylation; phosphorylation or other such modifications.

If the target is an antibody, the antibody may be selected from one or more of the classes IgA, IgD, IgE, IgG and IgM.

If the target is a nanoparticle, the nanoparticle can be selected from, but is not limited to, one or more of insulating, metallic or semiconducting nanoparticles.

If the target is a drug, the drug may be selected from, but is not limited to, alcohol (e.g. ethanol), amphetamines, amyl nitrate, heroin, ketamine, anabolic steroids, LSD, solvents, cannabis, cocaine (such as cocaine hydrochloride or 'coke'), tobacco, tranquilisers, crack (i.e. cocaine free base), ecstasy and/or gammhydroxybutyrate (GHB). Alternatively, in some embodiments, the drug may be a medicinal substance.

The target may be a candidate drug, e.g. a chemical or biological entity which may be tested or screened for a particular activity or property using the present invention.

If the target is a toxin, the toxin may be selected from, but is not limited to, one or more toxins originating from animals, plants, or bacteria.

If the target is a viral particle, the viral particle may be selected from, but is not limited to, one or more viral particles with and without a genome.

If the target is a cell, the cell may be selected from, but is not limited to, one or more of pluripotent progenitor cells, human cells (e.g. B-cells, T-cells, mast cells, phagocytes, neutrophils, eosinophils, macrophages, endothelial cells), cancerous cells (e.g. those originating from liver, cervical bone, pancreatic, colorectal, prostate, epidermal, brain, breast, lung, testicular, renal, bladder cancers), unicellular organisms of non-human origin, algae, fungi, bacteria, plant cells, parasite eggs, plasmodia and mycoplasma.

If the target is an organelle, the organelle may be selected from, but is not limited to, one or more of nucleus, mitochondria, Golgi apparatus, endoplasmic reticulum, lysosome, phagosome, intracellular membranes, extracellular membranes, cytoskeleton, nuclear membrane, chromatin, nuclear matrix and chloroplasts.

If the target is a lipid, the lipid may be selected from, but is not limited to, one or more of signalling lipids, structural lipids, phospholipids, glycolipids and fatty acids.

If the target is nucleic acid sequence, the nucleic acid sequence may be selected from, but is not limited to, one or more of DNA, cDNA, RNA, rRNA, mRNA, miRNA and tRNA.

If the target is an oligosaccharide, the oligosaccharide may be selected from, but is not limited to, one or more of oligosaccharides of human, animal, plant, fungal or bacterial origin.

In a preferred embodiment, the target is a protein, which may be a glycoprotein. The method and other aspects of the invention may be used for the detection or identification of a protein.

The target may be any antigen or analyte that is indicative of a particular disease. The target may be selected from, for example, carcinoembryonic antigen (CEA), angiotensin I converting enzyme (peptidyl-dipeptidase A) 1; adiponectin; advanced glycosylation end product-specific receptor; alpha-2-HS-glycoprotein; angiogenin, ribonuclease, RNase A family, 5; apolipoprotein A-I; apolipoprotein B (including Ag(x) antigen); apolipoprotein E; BCL2-associated X protein; B-cell CLL/lymphoma 2; complement C3; chemokine (C-C motif) ligand 2; CD 14, soluble; CD 40, soluble; cdk5; pentraxin-related; cathepsin B; dipeptidyl peptidase IV; Epidermal growth factor; endoglin; Fas; fibrinogen; ferritin; growth hormone 1; alanine aminotransferase; hepatocyte growth factor; haptoglobin; heat shock 70kDa protein 1 B; intercellular adhesion molecule 1; insulin-like growth factor 1 (somatomedin C); insulin-like growth factor 1 receptor; insulin-like growth factor binding protein 1; insulin-like growth factor binding protein 2; insulin-like growth factor-binding protein 3; interleukin 18; interleukin 2 receptor, alpha; interleukin 2 receptor, beta; interleukin 6 (interferon, beta 2); interleukin 6 receptor; interleukin 6 signal transducer (gp130, oncostatin M receptor); interleukin 8; activin A; leptin (obesity homolog, mouse); plasminogen activator, tissue; proopiomelanocortin (adrenocorticotropin/ beta-lipotropin/ alpha-melanocyte stimulating hormone/ beta-melanocyte stimulating hormone/ beta-endorphin); proinsulin; resistin; selectin e (endothelial adhesion molecule 1); selectin P (granule membrane protein 140kDa, antigen CD62); serpin peptidase inhibitor, clade E (nexin, plasminogen activator inhibitor type 1), member 1; serum/glucocorticoid regulated kinase; sex hormone-binding globulin; transforming growth factor, beta 1 (Camurati-Engelmann disease); TIMP metallopeptidase inhibitor 2; tumor necrosis factor receptor superfamily, member 1 B; vascular cell adhesion molecule 1 (VCAM-1); vascular endothelial growth factor; Factor II, Factor V, Factor VIII, Factor IX, Factor XI, Factor XII, F/fibrin degradation products, thrombin-antithrombin III complex, fibrinogen, plasminogen, prothrombin, and von Willebrand factor, C-reactive protein and the like. The target may be derived from a human.

Also described is an apparatus comprising an electron spin resonance spectrometer, the apparatus adapted to obtain an electron spin resonance signal of a mixed sample, the mixed sample formed by contacting a probe molecule capable of binding selectively to a target with a first sample that may or may not contain the target,

wherein a paramagnetic moiety is bound to the probe molecule via two thioether linkages;

wherein the apparatus can detect the presence and, optionally, the concentration of the target in the mixed sample wherein the apparatus comprises the mixed sample. Preferably, an electron spin resonance signal of the probe molecule when bound to the target is different from an electron spin resonance signal of the probe molecule that is not bound to the target. The apparatus may detect the presence of the target by detecting a difference between the electron spin resonance signal of the mixed sample and the electron spin resonance signal a reference sample containing the probe molecule, but lacking the target. The apparatus may calculate the concentration of the target in the mixed sample by using a predetermined relationship between the concentration of the target and the electron spin resonance signal of the probe molecule. The predetermined relationship may be a relationship between the mean square deviation of electron spin resonance signal of a probe molecule bound to the target (the deviation being from the probe molecule that is not bound to the target, which may be in a reference sample lacking the target, as described herein), and the concentration of the target in a reference sample. The apparatus may be adapted to carry out the method described herein, and may comprise a computer for automation of any part of the process and/or any comparing and calculation to be carried out. The method and apparatus of the invention may use a microwave or optical electron spin resonance technique. Optical ESR has been found to increase sensitivity. Optionally, ferroelectric inserts may be present in the mixed sample and, optionally, any of the other samples. Ferroelectric inserts have also been found to increase the sensitivity of the method.

Also described is a molecule of the formula (III)

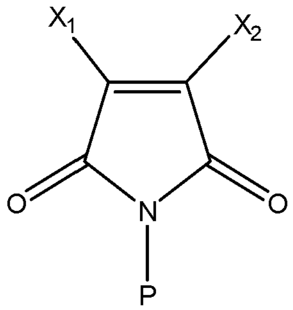

formula (III),

wherein X1 and X2 are each leaving groups, and P is a paramagnetic moiety. P may be a paramagnetic moiety as described herein. The leaving groups X1 and X2 may be any groups capable of being displaced by a thiol, such that the sulphur of the thiol, after displacement of the leaving group, forms part of a thioether linkage binding to the fivemembered maleimide ring. The thiol may be that of a cysteine amino acid or residue, which may optionally form part of a probe molecule, which may be as described herein.

X1 and X2 are independently selected from a halide, OR, SeR and SR, wherein R in any of OR, SR and SeR is a hydrocarbon group, and P is a paramagnetic moiety. Optionally R in OR, SR and SeR is a hydrocarbon group selected from an optionally substituted aryl, an optionally substituted heteroaryl, and an optionally substituted alkyl. Optionally X1 and X2 are both halide, both SR or both OR, wherein R is an optionally substituted aryl or heteroaryl. Aryl may be selected from, for example, phenyl and naphthyl. The molecule of formula (III) may be used to produce a probe molecule as described herein. P may be a paramagnetic moiety of formula (IV) or (V) above, in which L' links to N in formula (III). P may be a paramagnetic moiety of formula (IV) or (V) above, with all constituents in formula (IV) or (V) being as defined above, except that L' links to N in formula (III). P may be a paramagnetic moiety of formula (IV) or (V) above, in which L' is a single bond or $CH_2$ group linking to N in formula (III). The halide may be selected from chloride, bromide and iodide. R in SR, OR or SeR may be an optionally substituted aryl, optionally substituted heteroaryl, optionally substituted alkyl, optionally substituted alkenyl and optionally substituted alkynyl. R may be aryl selected from optionally substituted phenyl and optionally substituted naphthyl.

The term "aryl" as used herein may denote an optionally substituted monocyclic or polycyclic-aromatic group comprising carbon and hydrogen atoms. Examples of suitable aryl groups include, but are not limited to, phenyl and naphthyl (e.g. 1-naphthyl or 2-naphthyl). Suitable substituents for aryl are selected from the group consisting of $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $C_{1-6}$ alkylthio, arylthio, alkoxycarbonyl, amino, substituted amino for example alkylamino, carbamoyl, carbamate, ureido, amidino, imino, aryl, nitro, halogen and cyano. Optionally substituted phenyl can be for example 2-phenoxyphenyl, 2-methyl-phenyl, 3-methyl-phenyl, 4-methyl-phenyl, 2,3-dimethylphenyl, 2,4-dimethylphenyl, 2,5-dimethylphenyl, 2,6-dimethylphenyl, 3,4-dimethylphenyl, 3,5-dimethylphenyl, 2,3,4-trimethylphenyl, 3,4,5-trimethylphenyl, 2,3,4,5,6-pentamethylphenyl, 2-cyano-phenyl, 3-cyano-phenyl, 4-cyano-phenyl, 2,3-dicyanophenyl, 2,4-dicyanophenyl, 2,5-dicyanophenyl, 2,6-dicyanophenyl, 3,4-dicyanophenyl, 3,5-dicyanophenyl, 3,6-dicyanophenyl, 2-methoxyphenyl, 3-methoxyphenyl, 4-methoxyphenyl, 2,3-dimethoxyphenyl, 2,4-dimethoxyphenyl, 2,5-dimethoxyphenyl, 2,6-dimethoxyphenyl, 3,4-dimethoxyphenyl, 3,5-dimethoxyphenyl, 3,6-dimethoxyphenyl.

The term "heteroaryl" as used herein may mean a monocyclic or bicyclic radical of 5 to 12 ring atoms having at least one aromatic ring containing four to eight atoms per ring, incorporating one or more N, O, or S heteroatoms, the remaining ring atoms being carbon, with the understanding that the attachment point of said heteroaryl radical will be on said aromatic ring. As well known to those skilled in the art, heteroaryl rings have less aromatic character than their all-carbon counter parts. Thus, for the purposes of the invention, a heteroaryl group need only have some degree of aromatic character.

Examples of heteroaryl moieties include monocyclic aromatic heterocycles having 5 to 6 ring atoms and 1 to 3 heteroatoms including but not limited to, pyridinyl, pyrimidinyl, pyrazinyl, pyridazinone, pyrrolyl, pyrazolyl, imidazolyl, triazolinyl, thiophenyl and oxadiaxolinyl which can optionally be substituted with one or more, preferably one or two substituents selected from hydroxy, cyano, alkyl, alkoxy, thio, lower haloalkoxy, alkylthio, halo, haloalkyl, alkylsulfinyl, alkylsulfonyl, halogen, amino, alkylamino, dialkylamino, aminoalkyl, alkylaminoalkyl, dialkylaminoalkyl, nitro, alkoxycarbonyl and carbamoyl, alkylcarbamoyl and dialkylcarbamoyl and aryloxy such as benzyloxy.

The term "alkyl" as used herein may denote an unbranched or branched chain, saturated, monovalent hydrocarbon residue containing 1 or more carbon atoms. Examples of alkyl groups include, but are not limited to, lower alkyl groups include methyl, ethyl, propyl, *i*-propyl, n-butyl, *i*-butyl, *t*-butyl or pentyl, isopentyl, neopentyl and hexyl. For example, alkyl may refer to lower alkyl groups of $C_1$-$C_6$.

The term "substituted alkyl" as used herein denotes an alkyl group containing one or more substituents selected

from the group consisting of alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino, alkyl amino, dialkylamino, arylamino, diarylamino, alkylarylamino, acylamino, alkylcarbonylamino, arylcarbonylamino, carbamoyl, ureido, amidino, imino, sulfhydryl, alkylthio, arylthio, thiocarboxylate, sulfate, alkylsulfinyl, sulfonato, sulfamoyl, sulfonamido, nitro, trifluoromethyl, azido, heterocyclyl, alkylaryl or an aromatic or heteroaromatic group.

The molecule of formula (III) may be selected from any one of the following molecules

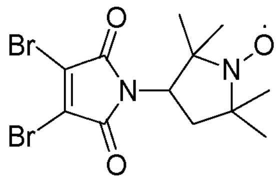

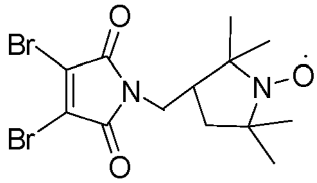

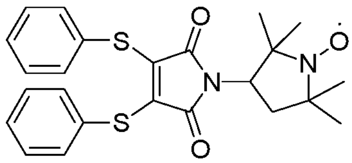

and

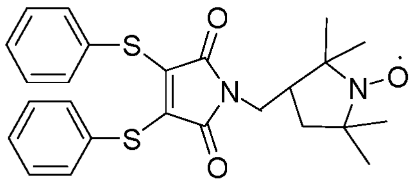

.

## Examples

The examples below are offered as an illustration and not by way of limitation.

A general method will first be described, followed by a specific, non-limiting Example. Any part of this general method may be combined with any of the aspects described herein and/or any other optional or preferred features described herein. As described in the Examples below, the method uses: i) a cystine bridged nitroxide spin label coupled sscFv (cystine stabilised single chain variable fragment) and ii) a solution that requires testing for the presence of a particular antigen of interest. As described above, an analogously modified antibody or other antibody fragment could also be used.

A binding signal is generated when complementation occurs fulfilled by prerequisites for the antigen and sscFv through the formation of high affinity non-covalent interactions. These take place through the amalgamation of weak interactions, such as: electrostatic (coulombic), Van der Waals, hydrophobic effects and hydrogen bonds. Once these prerequisites are satisfied, the sscFv is bound to its antigen and has a different EPR spectrum to its unbound form. This change can be attributed to the slowing of the tumbling time of the sscFv, as it forms a complex with its specific antigen. The sensitivity of the spectral change and formation of sscFv-antigen complexes is described by the law of mass action[21].

In carrying out the assay, a final concentration of modified sscFv and the finalised working volume of the entire solution is chosen and held constant. The antibody concentration is chosen to be large enough to be detected by the EPR spectrometer (in the test experiments where detection was achieved using a standard microwave resonator rather than optical methods, a minimum of ~100nM) and should not be so large that a binding signal cannot be seen at low antigen concentration (however, this is dictated by affinity of the system being investigated). A known amount of antigen

is then titred and the normalisation volume is added e.g. phosphate buffered saline (PBS) pH 7.4 (137mM NaCl, 2.7mM KCl, 100mM $Na_2HPO_4$, 2mM $KH_2PO_4$). To this mixture, the spin labelled sscFv is added at a known final concentration. This method can be used to generate a binding curve, from which the unknown antigen concentration of test samples can be estimated according to their mean square deviation (MSD) from the unbound-free spectrum.

The method was found to be dependent on the binding constant of the respective antibody and antigen pair, concentration of labelled sscFv available, avidity of the sscFv and the dynamic range of the antigen concentration being investigated.

The matrix (i.e. carrier medium) within which the measurement is taken e.g. whole blood, plasma, buffer, spinal fluid etc. is used to generate the standard binding curve if required. This is to evaluate the cross reactivity within the test samples of any housekeeping biomolecules as they must be acknowledged if present, especially if there is an impact on the sscFv EPR spectrum in the absence of antigen. This includes the incorporation of any pre-treatments of the samples, e.g. the addition of 109 mM sodium citrate as whole blood anticoagulant.

The solutions are mixed for a reasonable period of time until equilibrium is reached; temperature can also be controlled within the EPR resonator to obtain thermodynamic binding energies for the antibody-antigen complexes of interest.

The current working volumes can be anywhere between 10-50 $\mu$l depending on the available concentration of the sscFv reagent, the dynamic range of the antigen, the EPR spectrometers microwave frequency and cavity dimensions. Ferroelectric inserts allowing for working volumes of less than 1 $\mu$L sample volumes for biosensing[22], and optical detection[16] of paramagnetic resonance opens the possibility of sub-nanolitre volumes.

The pH and salt concentration within the mixtures will vary widely depending on the matrix within which the antigen is found e.g. human plasma, blood, wastewater, etc.

The order of addition of each reagent can be as follows: the normalisation volume of buffer is added to the matrix containing the antigen and mixed to homogeneity; to this the sscFv is supplemented at a known final concentration and mixed to homogeneity until equilibrium is reached. The order of addition is not crucial as long as the generation of the calibration curve is unbiased.

The sscFv EPR spectrum undergoes free sscFv to bound sscFv spectrum changes upon the addition of antigen. The qualitative change is translated into a quantitative change by taking the mean squared deviation of each antigen titration EPR spectrum. A single titre represents a single data point towards a binding curve, from which thermodynamic binding constants can be extracted. The EPR spectra are normalised to their maximum and -1 0 +1 nitroxide (N = 1) transitions must be aligned before computing their mean squared deviations as described in equation 1.

$$\frac{1}{N}\sum(Xc - Xo)^2 \qquad \text{Eq. (1)}$$

Where N is the number of data points in the EPR spectrum, *Xc* is the spectrum containing antigen at concentration c where c→∞, Xo is the spectrum containing no antigen i.e. c→0.

For the most part, sscFv are the primary molecules whose spectrum is followed and mean squared deviation monitored as a function of antigen concentration. Whole antibodies can also be used utilised using the same modification strategies.

Data are typically fit using the following equation derived from the law of mass action:

$$fB = \frac{([L]+[Bi]+Kd)-\sqrt{(([L]+[Bi]+Kd)^2-4.[L].[Bi])}}{2.[Bi]} \qquad \text{Eq. (2)}$$

Where *fB* is the fraction bound, [*L*], ligand concentration, [*Bi*], initial antibody concentration, *Kd,* dissociation constant.

An alternative approach, utilising beads or a solid support to immobilize antigen by adsorbing the antigen on to the beads and then measuring the EPR spectrum of the bound whole antibody could be employed. The amount of label on the antibody bound to the antigen immobilised on the bead would be known, this way the amount of antibody bound to the bead is known and in parallel so is the antigen concentration. Using a solid support also concentrates the spin labelled material resulting in indirect signal amplification of the bead-antigen-antibody bound antibody spectrum. Whole cells presenting antigen can also replace the bead-bound-antigen solid support for whole cell EPR sensing.

Preparation of Materials

Example 1

*Synthesis of Anti-CEA sscFv utilised in the experiments below*

A DNA fragment encoding shMFE was synthesized (Genescript USA Inc.) and contains the two substitutions at Gly'H44'Cys as well as Ala'L100'Cys (Kabat numbering), the linker was extended from $(Gly_4Ser)_3$ (SEQ ID NO: 2) to $(Gly_4Ser)_4$ (SEQ ID NO: 3) and sequences encoding KREAEA (SEQ ID NO: 4) and His6 (SEQ ID NO: 5) *plus* stop codon were added to the 5 and 3 prime, respectively. The sequence was digested with EcoRI and *Not*I, and ligated into a pPICZαB vector (Invitrogen). After amplification in *E. coli* TOP10 (NEB) the construct was linearized with *PmeI* and transferred by electroporation into *Pichia pastoris* X33 (Invitrogen). We used the transformant with the highest protein expression for protein production by fermentation with initial purification using radial bed adsorption IMAC as published[24, 25]. The IMAC fraction containing the protein was concentrated with a Labscale TFF system (Millipore) using a Biomax 10 kDa ultrafiltration module and subsequently applied to a Superdex 75 size-exclusion column (500 ml bed volume, GE Healthcare) which was equilibrated with PBS (pH 7.4). The fraction containing the monomer sscFv was collected and stored at -80 °C. The amino acid sequence of the resultant anti-CEA sscFv is shown below. Cysteines forming the readily modifiable disulfide bridge are underlined in bold text.

We synthesized and purified the NA1 domains of CEA as described elsewhere[26].

**1**QVKLEQSGAEVVKPGASVKLS**C**KASGFNIKDSYMHWLRQGPGQ**C**LEWIGWIDPENGDTEYAPKFQ

**66** GKATFTTDTSANTAYLGLSSLRPEDTAVYY**C**NEGTPTGPYYFDYWGQGTLVTVSSGGGGSGGGGSG

**131** GGGSGGGGSENVLTQSPSSMSASVGDRVTIA**C**SASSSVPYMHWFQQKPGKSPKLLIYSTSNLASGVP

**197** SRFSGSGSGTDYSLTISSVQPEDAATYY**C**QQRSSYPLTFG**C**GTKLEIK (SEQ. ID. NO. 1)

Example 2

*Synthesis of NdiTPMSL (Dithiophenolmaleimide-N-PROXYL spin label [TPMP]) and CNdiTPMSL (Dithiophenolmaleimide-N-methyl-PROXYL spin label* [*TPM*$_c$*P*])

3,4-Dibromo-2,5-dioxo-2,5-dihydro-pyrrole-1-carboxylic acid methyl ester

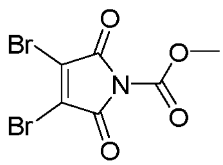

To dibromomaleimide (1.0 g, 3.9 mmol) and N-methyl morpholine (433 μl, 3.9 mmol) in THF (35 ml) was added methyl chloroformate (304.0 μl, 3.9 mmol). The reaction was stirred for 20 min at ambient temperature. The solid was dissolved in dichloromethane (40 ml) and the solution was washed with water (4x 50 ml), dried with sodium sulphate and the solvent removed *in vacuo* to yield the product as a pink powder (1.03 g, 84%).

$^1$H NMR (500 MHz, $CDCl_3$): δ = 4.00 (s, 3H, $CH_3$); $^{13}$C NMR (125 MHz, $CDCl_3$): δ = 159.4 (C), 147.1 (C), 131.6 (C), 55.0 ($CH_3$); IR (solid, cm$^{-1}$): 3020 (s), 1780 (m), 1735 (m); MS (CI) *m/z,* (%): 315 ($^{81,81}$M+, 8), 313 ($^{79,81}$M+, 19), 311 ($^{79,79}$M, 11), 255 (59), 205 (100); Mass calc. for $CrH_3O_4N^{79}Br_2$: 310.8423. Found: 310.8427; m.p. 156-158 °C.

**Dibromomaleimide-N-PROXYL spin label**

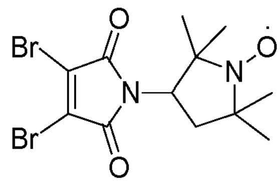

To 3,4-Dibromo-2,5-dioxo-2,5-dihydro-pyrrole-1-carboxylic acid methyl ester (441.9 mg, 1.9 mmol) in DCM (20 ml) was slowly added 3-amino-2,2,5,5-tetramethyl-1-pyrrolidinyloxy (144.0 mg, 0.9 mmol) in DCM (5 ml) and the reaction was stirred for 60 min at ambient temperature. The solvent was removed *in vacuo* and the residual material was purified by flash chromatography on silica gel (methanol:dichloromethane, gradient elution from 0.0 to 4.0%) to afford the product as an impure mixture. The material was re-purified by flash chromatography on silica gel (petroleum ether:diethyl ether, gradient elution from 9:1 to 7:3) to afford the product as a brown powder (186 mg, 50%).

$^1$H NMR (500 MHz, $CDCl_3$, crude data after treatment with hydrazobenzene*): δ = 4.51 (dd, H, *J=11.3,* 8.8, CH), 2.92 (appt. t, H, *J=12.2,* $CH_2$), 1.84 (dd, H, *J=12.9, J=8.7,* $CH_2$), 1.38 (s, 3H, $CH_3$), 1.28 (s, 3H, $CH_3$), 1.26 (s, 3H, $CH_3$), 1.11 (s, 3H, $CH_3$); $^{13}$C NMR (125 MHz, $CDCl_3$, crude data after treatment with hydrazobenzene*): δ = 164.4 (C), 129.7 (C), 57.1 (CH), 35.7 ($CH_2$), 27.2 (C), 25.6 (C), 25.2 (2x $CH_3$), 21.6 (2x $CH_3$); IR (solid, $cm^{-1}$): 2975 (w), 2934 (w), 1785 (w), 1722 (s), 1603 (m); MS (EI) *m/z,* (%): 397 ($^{81,81}$M+, 12), 395 ($^{79,81}$M+, 22), 393 ($^{79,79}$M, 12), 309 (100); Mass calc. for $C_{12}H_{15}O_3N_2{}^{79}Br_2$: 392.9444. Found: 392.9440; m.p. 171-176 °C.

*In order to obtain detailed NMR spectra it was necessary to reduce the PROXYL radical prior to NMR-spectroscopy. This was done by treatment of the purified spin label with a 3x excess (by mass) of hydrazobenzene for 60 min. The presented data is thus an excerpt of the mixture of the spin label, hydrazobenzene and their reaction products.

**Dithiophenolmaleimide-N-PROXYL spin label (TPMP)**

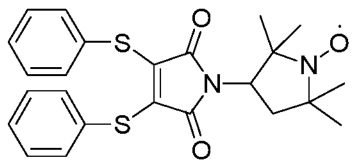

To the dibromomaleimide-N-PROXYL spin label (100.0 mg, 0.3 mmol) and sodium hydrogencarbonate (107.1 mg, 1.3 mmol) in methanol (35 ml) was slowly added benzenethiol (52.2 μl, 0.6 mmol) in methanol (5 ml). The reaction was stirred for 10 min at ambient temperature. The solvent was removed *in vacuo* and the residual material was purified by flash chromatography on silica gel (petroleum ether:ethyl acetate, gradient elution from 9:1 to 7:3) to afford the product as an orange powder (79 mg, 68%).

$^1$H NMR (500 MHz, $CDCl_3$, crude data after treatment with hydrazobenzene*): δ = 7.35-7.19 (m, 10H, Ar-H), 4.29 (dd, H, *J=8.9,* 5.3, CH), 3.00 (dd, H, *J=10.2,* 7.6, $CH_2$), 2.04 (dd, H, *J=12.5, J=7.4,* $CH_2$), 1.31 (s, 3H, $CH_3$), 1.23 (s, 3H, $CH_3$), 1.21 (s, 3H, $CH_3$), 1.08 (s, 3H, $CH_3$); $^{13}$C NMR (125 MHz, $CDCl_3$, crude data after treatment with hydrazobenzene*): δ = 167.4 (C), 136.0 (C), 132.4 (CH), 131.9 (CH), 131.1 (CH), 129.4 (C), 56.3 (CH), 35.5 ($CH_2$), 27.3 (C), 25.7 (C), 24.9 (2x $CH_3$), 21.9 (2x $CH_3$); IR (solid, $cm^{-1}$): 2973 (w), 2930 (w), 1771 (w), 1708 (s), 1631 (w), 1582 (w), 1537 (w); MS (EI) *m/z,* (%): 453 (M, 17), 99 (100); Mass calc. for $C_{24}H_{25}O_3N_2S_2$: 453.1307. Found: 453.1292; m.p. 124-128 °C.

**Dibromomaleimide-N-methyl-PROXYL spin label**

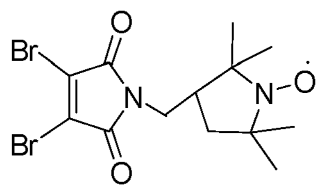

To 3,4-Dibromo-2,5-dioxo-2,5-dihydro-pyrrole-1-carboxylic acid methyl ester (274.6 mg, 0.9 mmol) in DCM (20 ml) was slowly added 3-(aminomethyl)-PROXYL (75.0 mg, 0.4 mmol) in DCM (3 ml) and the reaction was stirred for 24 h at ambient temperature. The solvent was removed *in vacuo* and the residual material was purified by flash chromatography on silica gel (petroleum ether:diethyl ether, gradient elution from 9:1 to 0:1) to afford the product as a brown powder (132 mg, 73%).

$^1H$ NMR (500 MHz, $CDCl_3$, crude data after treatment with hydrazobenzene*): δ = 3.68 (dd, H, *J=13.8,* 4.9, CHH), 3.53 (dd, H, *J=14.6, 10.3,* CH*H*), 2.17-2.01 (m, H, CH), 1.70 (dd, H, *J=12.8, J=7.7,* $CH_2$), 1.60 (appt. t, H, *J=11.7,* $CH_2$), 1.22 (s, 6H, 2x$CH_3$), 1.15 (s, 3H, $CH_3$), 1.09 (s, 3H, $CH_3$); $^{13}C$ NMR (125 MHz, $CDCl_3$, crude data after treatment with hydrazobenzene*): δ = 164.0 (C), 129.6 (C), 42.5 (CH), 40.9 ($CH_2$), 40.4 ($CH_2$), 28.2 (C), 26.3 (C), 26.2 (2x $CH_3$), 25.7 (2x $CH_3$); IR (solid, $cm^{-1}$): 2971 (w), 2933 (w), 1784 (w), 1718 (s), 1696 (m); MS (EI) *m/z,* (%): 411 ($^{81,81}$M+, 21), 409 ($^{79,81}$M+, 46), 407 ($^{79,79}$M, 21), 323 (100), 308 (36), 268 (58); Mass calc. for $C_{13}H_{17}O_3N_2{}^{79}Br_2$: 406.9600. Found: 406.9596; m.p. 136-139 °C.

**Dithiophenolmaleimide-N-methyl-PROXYL spin label (TPMcP)**

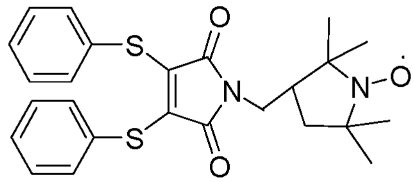

To the dibromomaleimide-N-methyl-PROXYL spin label (80.0 mg, 0.2 mmol) and sodium hydrogencarbonate (82.2 mg, 1.0 mmol) in methanol (30 ml) was slowly added benzenethiol (40.1 μl, 0.4 mmol) in methanol (3 ml). The reaction was stirred for 10 min at ambient temperature. The solvent was removed in *vacuo* and the residual material was purified by flash chromatography on silica gel (petroleum ether:ethyl acetate, gradient elution from 9:1 to 7:3) to afford the product as a viscous yellow liquid (44 mg, 47%).

$^1H$ NMR (500 MHz, $CDCl_3$, crude data after treatment with hydrazobenzene*): δ = 7.33-7.26 (m, 10H, Ar-H), 3.57 (dd, H, *J=13.8,* 5.2, CHH), 3.41 (dd, H, *J=14.2, 4.3,* CHH), 2.16-2.09 (m, H, CH), 1.65 (dd, 2H, *J=12.4, J=7.7,* $CH_2$), 1.55 (appt. t, H, *J=11.8,* $CH_2$), 1.18 (s, 3H, $CH_3$), 1.16 (s, 3H, $CH_3$), 1.12 (s, 3H, $CH_3$), 1.02 (s, 3H, $CH_3$); $^{13}C$ NMR (125 MHz, $CDCl_3$, crude data after treatment with hydrazobenzene*): δ = 166.8 (C), 136.0 (C), 132.0 (CH), 131.1 (CH), 129.4 (C), 128.6 (CH), 41.9 (CH), 40.9 ($CH_2$), 39.9 ($CH_2$), 29.1 ($CH_3$), 26.6 ($CH_3$), 26.5 ($CH_3$), 23.9 ($CH_3$); IR (solid, $cm^{-1}$): 2971 (w), 2932 (w), 1737 (w), 1704 (s), 1581 (w); MS (EI) *m/z,* (%): 467 (M, 44), 326 (55), 161 (47), 110 (100) Mass calc. for $C_{25}H_{27}O_3N_2S_2$: 467.1458. Found: 467.1462.

## Example 3

### *Anti-CEA sscFv TPMP and TPMcP Modification*

A solution of the CEA specific sscFv in PBS (pH 7.4) was diluted in the same buffer and dimethylformamide (DMF, 10% vol/vol final amount) to yield a concentration of 70.0 μM (1.87 mg $ml^{-1}$) prior to experimentation. All maleimides and benzeneselenol were prepared in DMF as highly concentrated stock solutions. 2 equiv TPMP or TPMcP were added to the antibody solution followed by 25 equiv of benzeneselenol. The mixture was incubated for 20 min at ambient temperature. Functionalized antibody fragments were purified with PD MiniTrap G-25 desalting columns (GE Healthcare) and concentrated using concentrators with 5KDa MWCO (Sartorius). Concentrations were determined by UV/Vis using a calculated molecular extinction coefficient of $\varepsilon_{280}$ = 48,735 $M^{-1}$ $cm^{-1}$. Successful modification was confirmed by LC-MS and EPR. EPR was also used to confirm labeling efficiencies.

## Carrying out the Analytical Method

## Example 4 - Saturation Binding Curves

A known concentration of purified antigen N-A1 (carcinoembryonic antigen fragment, comprising two of the N-terminal immunoglobulin domains of the carcinoembryonic antigen - molecular weight ~26kDa) is added to human plasma at a final concentration ranging from 0 to saturation within a normalised final volume i.e. 15μl. To the mixture we add a known concentration of spin labelled anti-CEA (anti-carcinoembryonic antigen) sscFv specific for the antigen.

(The free EPR spectrum of the sscFv in the absence of antigen is known). The mixture is homogenized using a thermomixer at ~1Krpm under a controlled temperature of 37°C specific for the assay for 30minutes. The sample is then centrifuged into the bottom of a flame sealed 50μL micropipette sample holder using a cushioned support for ~1 seconds at 1 Krpm. The sample is then transferred to a temperature controlled EPR cavity and the EPR spectra are recorded. After all data points have been acquired, mean squared deviations are computed and a standard curve is generated. The curve can be used to acquire thermodynamic binding constants and can be used as a reference to compare unknown antigen concentration. Acquired data can also be simulated.

**Example 5 - Thermal Denaturation**

The temperature control unit offset was calibrated using a thermocouple bound to a 15 μL loaded quartz capillary. 10μM anti-CEA alone and in bound form (i.e. 10 μM anti-CEA saturated with 70 μM N-A1) in 15μL final volume was heated from -20 °C - -75 °C. The N-A1 acted to stabilise and delay the rate at which anti-CEA denatured or tumbled when compared to anti-CEA denaturation alone. The experiment was also performed for a higher affinity sscFv and showed a similar but greater stabilizing effect of -10K when compared to its lower affinity analogue. The sample was heated through the convection of heated oxygen free nitrogen gas.

**Example 6 - Real-Time Monitoring**

Two columns of solution were formed at one end of a 1mm I.D. capillary of a flame sealed tube. The first column at the bottom of the tube contains 20 μM of anti-CEA sscFv and the second column formed above it using an extruded glass Pasteur pipette contains 70 μM NA1. A sealed extruded Pasteur pipette was then used as a plunger to mix the two columns together within the ESR spectrometer's resonator during a single scan. This scan time was known to be 26.01 secs where 12 mixing 'plunges' were performed thoroughly mixing both solutions. Data were then accumulated and indicated that a binding reaction was taking place and followed in real-time in solution for ~50mins. This binding event could be followed in a range of solutions, with the possibility of yielding equilibrium constants.

**Discussion of the Figures**

(All experiments were performed at conventional X-band microwave frequency).

**Figures 1(a) to 1(e) illustrate schematically an embodiment of a method of the invention.** In particular in these Figures, a) Shows a spin labelled sscFv in the form of spin labelled anti-CEA sscFv (green). The disulfide bond is located at the opposite end of the scFv. The modified sscFv as in a) is added to b), which is a matrix containing the antigen that is being detected; the mixture is then incubated if required, c), for a set time and temperature. d) a complex is formed during the incubation time comprising the modified sscFv in a) and antigen (blue). An EPR measurement is then taken as depicted in e), where the black trace is the EPR spectrum of a) alone and the grey trace is the EPR spectrum of the complex formed in d).

**Figures 2(a) to 2(d) illustrate binding constant acquisition using an embodiment of the invention.** In particular in these Figures: a) 10 μM sscFv MP-anti-CEA N-A1 binding curves (TPMP does not contain an extra carbon and is a rigid label with restricted endogenous mobility). N-A1 concentrations were 0, 3, 6, 9, 12, 15, 20, 22.5, 25, 30, 35, 40, 45, 50, 60, 70 μM. Black, light grey, dark grey solid lines represent PBS pH 7.4, human plasma and whole human blood best fit lines for raw data, where closed circles represent their respective data points. Dashed lines and open circles data points represent fits of simulated data using MATLAB plug-in Easyspin (code to generate binding curves is shown in Appendix A). Here, the simulated data represents the weightings of each component as a function of antigen concentration. b) same as a but for $M_c$P-anti-CEA (TP$M_c$P contains an extra carbon containing the between the cystine bridging agent and nitroxide ring, providing inherent label flexibility). c) 250 nM sscFv MP-anti-CEA N-A1 and FLCEA (full-length CEA) binding curves. Antigen concentrations were 0, 0.025, 0.05, 0.075, 0.1, 0.125, 0.1875, 0.25, .375, 0.5, 0.625, 0.75, 0.875, 1, 1.25, 1.5, 1.75 μM. Closed and open circles are the same as a) and annotations on the plots indicate the antigen type used. d) Same as c) but for McP-anti-CEA. Data were fit using Eq. 2, mentioned above.

**Table 1.1 shows binding constants and rotational correlation times (Tc) acquired using non-linear least squares fitting and Easyspin, respectively.** In particular, the table shows the matrix type, label type and sscFv concentration used in the respective experiments with the respective antigen. $Tc_{Mobile}$ provides the rotational correlation time used to simulate the mobile components and Tc Immobile provides the rotational correlation times used to simulate the immobile component - both in nanoseconds. Kd values from both raw data and simulated (fraction or weightings from simulated data) and the respective $R^2$ values from the fits are also shown.

***Table 1.1.*** - ¶ FLCEA was the antigen for these experiments. I I

| sscFv analogue : antigen | Matrix - sscFv concentration | aTc Mobile (ns) | bTc Immobile (ns) | Source of data | $K_d$ – (μM) | $R^2$ |
|---|---|---|---|---|---|---|
| MP-sscFv : NA1 | PBS - 10μM | 2.5818 | 28.148 | raw | 1.911 | 0.962 |
| | | | | simulation | 1.216 | 0.964 |
| | Serum - 10μM | 2.9882 | 14.9549 | raw | 4.352 | 0.956 |
| | | | | simulation | 3.938 | 0.880 |
| | Blood - 10μM | 1.0079 | 8.5125 | raw | 6.462 | 0.948 |
| | | | | simulation | 5.628 | 0.877 |
| | Blood - 0.25μM | 1.0079 | 8.5125 | raw | 0.148 | 0.939 |
| | | | | simulation | 0.110 | 0.909 |
| MP-sscFv : full length CEA | PBS - 10μM | 1.0079 | 8.5125 | raw | 0.064 | 0.811 |
| | | | | simulation | 0.052 | 0.948 |
| McP-sscFv : NA1 | PBS - 10μM | 0.8383 | 6.435 | raw | 5.621 | 0.872 |
| | | | | simulation | 3.718 | 0.863 |
| | Serum - 10μM | 1.011 | 6.4661 | raw | 7.956 | 0.928 |
| | | | | simulation | 5.422 | 0.929 |
| | Blood - 10μM | 0.932 | 7.7657 | raw | 6.824 | 0.919 |
| | | | | simulation | 5.589 | 0.903 |
| | Blood - 0.25μM | 1.0612 | 3.9829 | raw | 0.151 | 0.836 |
| | | | | simulation | 0.124 | 0.913 |
| McP-sscFv : full length CEA | Blood - 0.25μM | 1.0612 | 3.9829 | raw | 0.074 | 0.860 |
| | | | | simulation | 0.055 | 0.983 |

[a] $T_{C\ Mobile}$ shows the rotational correlation time estimated with Easyspin[1] and used to simulated weighting for simulated binding curves.
[b] $T_{C\ Immobile}$ is the same as [a] but for the simulated immobile component.

**Figures 3(a) to 3(t) show raw and simulated data acquired to generate Figure 2.** A-tensor values were: [$A_{xx}$ 22 $A_{YY}$ 23 $A_{zz}$ 94] MHz and g-tensor values were: [$g_{xx}$ 2.0087 $g_{YY}$ 2.0066 gzz 2.0026]. MSD were computed over the entire spectra including all data points and microwave frequency were ~9.7GHz (an X-band ESR spectrometer was used to collect all data). In particular, in these Figures:

**a)** 10 μM MP-anti-CEA N-A1 binding curve EPR spectra, N-A1 concentration increases from bottom to top, 0 - 70 μM as indicated by the gradient bar, in PBS pH 7.4.
**b)** Simulated spectra from acquired data a).
**c)** Same as **a)** but for McP-anti-CEA.
**d)** Same as **b)** but for acquired dataset **c).**
**e)** Same as **a)** but in human plasma.
**f)** Same as **b)** but for acquired dataset **e).**
**g)** Same as **e)** but for McP-anti-CEA.
**h)** Same as **b)** but for acquired dataset **g).**
**i)** Same as **a)** but in whole human blood.
**j)** Same as **b)** but for acquired dataset **i).**
**k)** Same as **i)** but for McP-anti-CEA.

**l)** Same as **b)** but for acquired dataset **k).**
**m)** 250 nM MP-anti-CEA N-A1 binding curve EPR spectra, N-A1 concentration increases from bottom to top, 0 - 1.75 μM as indicated by the gradient bar, in whole human blood.
**n)** Simulated spectra from acquired data **m).**
**o)** Same as **m)** but where the antigen is full length CEA.
**p)** Same as **n)** but for acquired dataset **o).**
**q)** Same as **m)** but for McP-anti-CEA.
**r)** Simulated spectra from acquired data **q)**
**s)** Same as **q)** but where the antigen is full length CEA.
**t)** Simulated spectra from acquired data **s).**

**Figures 4(a) to (h) illustrate biophysical analysis of anti-CEA complex using thermal denaturation - EPR Spectra.** In particular, in these Figures: **a)** 10 μM anti-CEA MP-anti-CEA unbound thermal denaturation spectra recorded at: 20, 25, 30, 35, 40, 45, 50, 52, 54, 56, 58, 60, 62, 65, 67, 70, 72, 75°C. b) Same as a) but with 70μM NA1. c) same as a) but using a higher affinity anti-CEA sscFv, SM3E. d) is the same a b) but utilises SM3E anti-CEA. e), f), g), h) are the same as a), b), c), d), respectively, but utilise TPMcP. All sample volumes were 15μL and were carried out in PBS pH 7.4.

**Figures 5(a) and 5(b) illustrate biophysical analysis of anti-CEA complex using thermal denaturation - denaturation profile of EPR spectra in Figure 4.** In particular, in these Figures: a) Thermal denaturation profile of MP-anti-CEA (including SM3E analogue) generated from Figures 4 a), b), c), d) as indicated by the figure legend. b) same as a) but for TPMcP and generated from Figures 4 e), f),g) h). Data represent the ratio of the low field and $G_{ISO}$ nitroxide peak. The shift of the inflection point to higher temperature is consistent with the bound state of the protein resulting in a smaller propensity to unfold.

**Figures 6(a) and (b) illustrate anti-CEA binding kinetics followed by EPR.** In particular, in these Figures: a) 20 μM anti-CEA and 70μM NA1 binding followed as a function of time. The green spot represent the starting amplitude of the low field nitroxide peak of the normalised EPR spectrum measured before mixing and is representative of its maximum in the unbound form; hence, it represents the starting point of the reaction. The red spot (denoted by 'Finish' in the legend in Figure 6(a)) represent the maximum of the low field peak when the reaction has reached completion. The curve is fit with a non-linear least square second order exponential decay which acts as a guide for the eye only. b) represents the EPR spectra utilised for monitoring the binding reaction in a). The difference between the green and blue trace represents the reaction spectra during the 26.01secs of dead time where mixing and binding has taken place. The annotations on the plot describe the other spectra. Microwave frequency was ~9.8GhZ. Here, a very crude mixing method was used but shows the scope of using real-time monitoring of antibody-antigen binding in solutions.

***Appendix A - MATLAB code utilised to generate simulated weightings for simulated binding curve generation. Easyspin v4.0.0. plug-in is required for code to work.***

**Brief description of code:**

The MATLAB code utilising Easyspin[23] v4.0.0. plug-in is used to load EPR spectra, subtract baseline, perform baseline correction and if necessary align spectra (alignment of spectra must be done manually and is not provided by the code). EPR spectra are then normalised to their maximum. EPR spectra are then interpolated if necessary (if there is variability in the number of data points acquired). The EPR spectrum containing no antigen (unbound) and saturated EPR spectrum (bound) are then simulated to extract EPR spectra of bound (Tc immobile) and unbound (Tc Mobile) components by varying their weighting, correlation time and line-widths by non-linear least squares fitting. These two components are then fit to all the acquired EPR spectra to extract the weightings of the bound and unbound spectra that make up each of the respective spectra using a non-linear least squares fit. Here the Levenberg-Marquardt algorithm is used and is built in function of MATLAB. This value is then plot against the antigen concentration and represents the simulated weightings or fraction bound or unbound (they are the inverse of one another).

**%% - Load EPR spectra, background subtraction, baseline correction**

clear all, close all, clc

cd(''); % Change to directory containing EPR spectra background = eprload('***'); % Loads background (insert filename in ***)
[ba a] = eprload('***'); % Loads EPR spectra and respective magnetic field (insert filename in ***)
a = a - background; % Subtracts background from spectrum original = [***]; % Place all EPR spectra into a matrix

(insert variables in ***)
original = basecorr(original,[1],[0]); % 0th order baseline correction of data

**%% - Interpolate data if number of points are not the same**

f = original; % EPR spectra are re-assigned to variable f
Bst = ba(1); % Assign starting value of magnetic field to variable Bst
Bd = ba(length(ba)); % Assign end value of magnetic field to variable Bd
bi = linspace(Bst,Bd,***); % Create an array with starting value Bst and end value Bd with number of points specified by ***
f = interpl(ba,f, bi); % Assigns interpolated spectra to variable f

**%% - Simulate bound and unbound components from EPR spectra**

f1 = [original(:,*) original(:,*)]; % Assigns first (unbound) and last (bound) spectra to variable f1. * represents matrix column in matrix original

% If interpolated spectra are used change original to variable f

[B_exp, spc-exp,params] = eprload('***'); % Assigns experimental magnetic field to variable B exp

% Assigns EPR spectrum to variable spc_exp

% Assigns experimental parameter to cell params

step = 1; % Assigns scalar value 1 to counter for for loop

while step <= 2 % While counter is less than or equal to 2

for f1 = f1; % For all spectra in f1 do the following as long as the while loop holds...

Exp.mwFreq = params.MWFQ*1e-9; % Uses experimental microwave frequency in GHz

Exp.nPoints = length(f1); % Uses experimental number of points

Exp.CenterSweep = [(params.XMIN+params.XWID*0.5)/10 params.XWID/10] + ***; % Uses experimental magnetic field parameters in militesla and adjust using offset ***

Exp.Harmonic = 1; % Use first harmonic

spc_exp= f1; % Assign current EPR spectrum to variable spc_exp

% Immobile component simulation starting values

SysN.Nucs = '14N'; % Simulation nucleus is nitrogen 14

SysN.lwpp = [0.2]; % Simulation peak to peak line width in militesla

SysN.g = [2.0087 2.0066 2.0026]; % Simulation anisotropic G factor

SysN.A = [22 23 94] % Simulation anisotropic hyperfine splitting constant in MHz

SysN.logtcorr = -8.4; % Simulation rotational correlation time in seconds i.e (1 * 10^x)

SysN.weight = 5; % Simulation component weighting

% Mobile component simulation starting values

SysA.Nucs = ’14N’; % Simulation nucleus is nitrogen 14

SysA.lwpp = [0.20]; % Simulation peak to peak line width in militesla

SysA.g = [2.008 2.006 2.003]; % Simulation anisotropic G factor

SysA.A = [22 23 94] % Simulation anisotropic hyperfine splitting constant in MHz

SysA.logtcorr = -8.98; % Simulation rotational correlation time in seconds i.e (1 * 10^x)

SysA.weight = 0.35; % Simulation component weighting

[Bsim,spc_sim] = chili(SysN,Exp); % Specifying simulation to take SysN parameters mentioned above and relate them to experimental - Use chili function

[B_sim,spc_sim2] = chili(SysA,Exp); % Specifying simulation to take SysN parameters mentioned above and relate them to experimental - Use chili function

FitOpt.Method = ’levmar fcn’; % Use Levenberg-Marquardt algorithm for fitting data

FitOpt.Scaling = ’lsql’; % Use least square fitting methodology with a 1st order baseline correction if required

Vary.lwpp = [le-6]; % Vary peak-to-peak line width in militesla by 1e-6

Vary.logtcorr = 0.1; % Vary rotation correlation time of protein by 0.1

Vary.weight = 0.1; % Vary weighting of component by 0.1

Vary2.lwpp = [le-6]; % Vary peak-to-peak line width in militesla by 1e-6

Vary2.logtcorr = 0.1; % Vary rotation correlation time of protein by 0.1

Vary2.weight = 0.1; % Vary weighting of component by 0.1

% Perform fitting

[ans, BestSpc] =

esfit(’chili’,spc exp,{SysN,SysA},{Vary,Vary2},Exp,[],FitOpt); %

Simulates fitting procedure using chili for spc exp EPR spectrum

% Uses System variable specified in SysN and SysA and varies them

% respective to Vary and Vary2

% Assigns simulated spectra as matrix BestSpc

% Assigns all information of the simulated spectra in cell ans

answerl(step,:) = ans; % Assigns ans in a cell specified by matrix m x n where m is specific by the value of step (m) and length of ans (n)

BBB = plot(B sim,BestSpc); % Plots simulated EPR spectra and assigns it to handle BBB

getme(1:***,step) = get(BBB,'YData'); % Assigns EPR spectra in to a matrix variable getme

% *** represents length of experimental spectrum i.e. length(f1)

step = step + 1; % Increases counter value

end % Ends for loop

end % Ends while loop

**%% - Simulates weightings of pre-simulated data in each of the EPR spectra**

**%% as a function of increasing antigen concentration**

f1 = original; % Assigns matrix containing all EPR spectra to variable f1

step = 1; % Assigns scalar value 1 to counter for for loop

while step <= *** % While counter is less than or equal to *** i.e. amount of spectra

for f = f1; % For all spectra in f1 do the following as long as the while loop holds...

Exp.mwFreq = params.MWFQ*1e-9; % Uses experimental microwave frequency in GHz

Exp.nPoints = length(f1); % Uses experimental number of points

Exp.CenterSweep = [(params.XMIN+params.XWID*0.5)/10 params.XWID/10] + ***; % Uses experimental magnetic field parameters in militesla and adjust using offset ***

Exp.Harmonic = 1; % Use first harmonic

spc_exp= f; % Assign current EPR spectrum to variable spc_exp

% Immobile component simulation starting values

SysN.Nucs = answer1{1,2}.Nucs; % Nuclei used in answerl for immobile component

SysN.lwpp = (answerl{1,2}.lwpp); % Peak-to-peak line width in militesla used in answerl for immobile component
SysN.g = (answerl{1,2}.g); % Anisotropic G factor used in answerl for immobile component
SysN.A = (answer1{1,2}.A); % Anisotropic hyperfine splitting constant in MHz used in answerl for immobile component
SysN.logtcorr = (answerl{1,2}.logtcorr); % Rotational correlation time used in answerl for immobile component
SysA.weight = (answer1{1,2}.weight); % Weighting used in answerl for immobile component
SysA.Nucs = answerl{2,1}.Nucs; % Nuclei used in answerl for mobile component
SysA.lwpp = (answerl{2,1}.lwpp); % Peak-to-peak line width in militesla used in answerl for mobile component
SysA.g = (answerl{2,1}.g); % Anisotropic G factor used in answerl for mobile component
SysA.A = (answerl{2,1}.A); % Anisotropic hyperfine splitting constant in MHz used in answerl for mobile component
SysA.logtcorr = (answerl{2,1}.logtcorr); % Rotational correlation time used in answerl for mobile component
SysA.weight = (answerl{2,1}.weight); % Weighting used in answerl for mobile component

FitOpt.Method = 'levmar fcn'; % Use Levenberg-Marquardt algorithm for fitting data

FitOpt.Scaling = 'lsql'; % Use least square fitting methodology with a 1st order baseline correction if required

Vary1.weight = 1; % Vary weighting of component by 1

Vary21.weight = 1; % Vary weighting of component by 1

[ans, BestSpc] =

esfit('chili',spc exp,{SysN,SysA},{Varyl,Vary21},Exp, [],FitOpt) ; % Simulates fitting procedure using chili for spc_expEPR spectrum

% Uses System variable specified in SysN and SysA and varies them % respective to Vary1 and Vary21

% Assigns simulated spectra as matrix BestSpc

% Assigns all information of the simulated spectra in cell ans

AAA = plot(B sim,BestSpc)'; % Plots simulated EPR spectra and assigns it to handle AAA

getmel(1:***,step) = get(BBB,'YData'); % Assigns EPR spectra in to a matrix variable getme1

% *** represents length of experimental spectrum i.e. length(f1)

answer(step,:) = ans; % Assigns ans in a cell specified by matrix m x n where m is specific by the value of step (m) and length of ans (n)

step = step + 1; % Increases counter value

end % Ends for loop

end % Ends while loop

**%% - Plot MSD**

C = original - free; % Unbound spectrum (free) is subtracted from all EPR spectra (original)
% the variable free can be generated using MATLAB repmat function D = C.^2; % C is squared
E = mean(D); % Mean of D is taken
hold on; plot(X,E,'ro'); % Plot E (MSD) against X

**%% - Plot simulated weightings of mobile and immobile component**

for vr = [1:***] % for 1 to amount of spectra: ***

ZZ3(:,vr) = answer{vr,1}.weight / (answer{vr,1}.weight + answer{vr,2}.weight); % Extract weight of mobile component and calculate its total fraction
WW3(:,vr) = answer{vr,2}.weight / (answer{vr,1}.weight + answer{vr,2}.weight); % Extract weight of immobile component and calculate its total fraction

end % End for loop
ZZ3 = ZZ3 - min(ZZ3); % Subtract minimum of ZZ3
ZZ3 = ZZ3./max(ZZ3); % Normalise ZZ3 to maximum
X = [***]; % Antigen concentration
hold on; plot(X, ZZ3,'bo'); % Plot ZZ3 (mobile) against X
WW3 = WW3 - min(WW3); % Subtract minimum of WW3
WW3 = WW3./max(WW3); % Normalise WW3 to maximum
X = [***]; % Antigen concentration
hold on; plot(X, WW3,'bo'); % Plot WW3 (immobile) against X

References mentioned herein or otherwise useful for background

1. - Mizuno,T.M.K.T.Y.O.M.T.T. - Metal-ion-dependent GFP emission in vivo by combining a circularly permutated green fluorescent protein with an engineered metal-ionbinding coiled-coil. -129, -11383 (/20).

2. - Katz,E.S.-H.-I.L.W. - Electrical contacting of glucose oxidase in a redox-active rotaxane configuration. - 43, -3300 (/20).

3. - Yao,C.Z.T.T.J.W.R.C.Q.C.M.Z.B.H.J.F.W. - Hybridization assay of hepatitis B virus by QCM peptide nucleic acid biosensor. - 23, -885 (/20).

4. Kawazumi,H. - Compact surface plasmon resonance (SPR) immunosensor using multichannel for simultaneous detection of small molecule compounds. -108, -796 (/20).

5. Jeschke,G. & Polyhach,Y. Distance measurements on spin-labelled biomacromolecules by pulsed electron paramagnetic resonance. Phys. Chem. Chem. Phys. 9, 1895-1910 (2007).

6. Klare,J.P. & Steinhoff,H.J. Spin labeling EPR. Photosynth. Res. 102, 377-390 (2009).

7. Sowa,G. - Site-directed spin labeling studies on nucleic acid structure and dynamics. - 82, -197 (/20).

8. - Czogalla,A.P.A.J.A.S.A. - Attaching a spin to a protein -- site-directed spin labeling in structural biology. - 54, -244 (/20).

9. Czogalla,A., Pieciul,A., Jezierski,A., & Sikorski,A.F. Attaching a spin to a protein -- site-directed spin labeling in structural biology. Acta Biochim. Pol. 54, 235-244 (2007).

10. Jakobsen,U., Shelke,S.A., Vogel,S., & Sigurdsson,S.T. Site-directed spin-labeling of nucleic acids by click chemistry: detection of abasic sites in duplex DNA by EPR spectroscopy. J. Am. Chem. Soc. 132, 10424-10428 (2010).

11. Landsberger,F.R., Lenard,J., Paxton,J., & Compans,R.W. Spin-labeled electron spin resonance study of the lipid-containing membrane of influenza virus. Proc. Natl. Acad. Sci. U. S. A 68, 2579-2583 (1971).

12. Columbus,L. & Hubbell,W.L. A new spin on protein dynamics. Trends Biochem. Sci. 27, 288-295 (2002).

13. - Oppenheim,S.B.G.R.V. - Relationship of rotational correlation time from EPR spectroscopy and protein-membrane interaction. -118, -139 (/19).

14. Golstein, A. Leute R. K. Ullman E. F. Patent Application; 3690834 - Ligand Determination With Spin Labeled Compounds By Receptor Displacement. 10-5-1978. United States.

15. Ullman,E.F. - Homogeneous immunoassays: Historical perspective and future promise. - 76, -788 (/19).

16. Kohler,J., Disselhorst,J.A.J.M., Donckers,M.C.J.M., Groenen,E.J.J., Schmidt,J., & Moerner,W.E. Magnetic resonance of a single molecular spin. Nature 363, 242-244 (1993).

17. Hsia,J.C. - Spin-labeling as a general method in studying antibody active site. -129, - & (/19).

18. Hsia,J.C. - Membrane immunoassay: principle and applications of spin membrane immunoassay. - 308, -48 (/19).

19. Timofeev,V.P. - Selective spin-labelling of an N-acetylneuraminic acid residue in the Fab-region oligosaccharide of immunoglobulin M. -17, -176 (/19).

20. Willan,K.J. - Specific spin labelling of the Fc region of immunoglobulins. - 80, -136 (/19).

21. Reverberi,R. - Factors affecting the antigen-antibody reaction. - 5, -40 (/20).

22. Nesmelov,Y.E., Surek,J.T., & Thomas,D.D. Enhanced EPR Sensitivity from a Ferroelectric Cavity Insert. Journal of Magnetic Resonance 153, 7-14 (2001).

23. Stefan Stoll, Arthur Schweiger. EasySpin, a comprehensive software package for spectral simulation and analysis in EPR. J. Magn. Reson. 178(1), 42-55 (2006)

24. Tolner, B., Smith, L., Begent, R.H. & Chester, K.A. Production of recombinant protein in Pichia pastoris by fermentation. Nat Protoc 1, 1006-1021 (2006).

25. Tolner, B., Bhaysar, G., Foster, B., Vigor, K. & Chester, K. in Laboratory Protocols in Fungal Biology. (eds. V.K. Gupta, M. Tuohy, A. Manimaran, K. Turner & R. Ovonvan) Chapter 37 (Springer Science and Business Media, New York; 2012).

26. Sainz-Pastor, N. et al. Deglycosylation to obtain stable and homogeneous Pichia pastoris-expressed N-A1 domains of carcinoembryonic antigen. Int J Biol Macromol 39, 141-150 (2006).

## Claims

**1.** An electron spin resonance (ESR) analytical method comprising:

providing a probe molecule capable of binding selectively to a target, wherein a paramagnetic moiety is bound to the probe molecule via two thioether linkages, wherein each thioether linkage is a carbon-sulphur-carbon linkage and one carbon of the thioether linkage is part of the probe molecule, and the other carbon is part of either the paramagnetic moiety or a linker group that binds to the paramagnetic moiety;
contacting the probe molecule with a first sample, which may or may not comprise the target, to form a mixed sample and obtaining an electron spin resonance signal of the mixed sample,

wherein the probe molecule is selected from an antibody fragment, an antibody and an aptamer.

**2.** An ESR analytical method according to claim 1, wherein the paragmagnetic moiety is bound to the probe molecule via two thioether linkages, wherein the sulphur atom in each thioether linkage forms part of a cysteine residue in the probe molecule.

**3.** An ESR analytical method according to claim 1 or claim 2, wherein the antibody is a single chain variable fragment.

**4.** An ESR analytical method according to any one of the preceding claims, wherein the probe molecule is derived from a cystine stabilised single chain variable fragment of an antibody, wherein the disulphide bond of the cystine has been cleaved and each sulphur atom of each cysteine bonded to the paramagnetic moiety as part of a thioether linkage, optionally via a linker moiety.

**5.** An ESR analytical method according to any one of the preceding claims, wherein the electron spin resonance signal of the mixed sample is compared with an electron spin resonance signal of the probe molecule that is not bound to the target, a difference in the signal indicating the presence of the target.

**6.** An ESR analytical method according to any one of the preceding claims, wherein the mixed sample contains the target and the concentration of the target in the mixed sample and/or the first sample is determined from the electron spin resonance signal of the mixed sample.

**7.** An ESR analytical method according to claim 6, wherein the concentration is determined by using a predetermined relationship between the mean square deviation of electron spin resonance signal of the probe molecule bound to the target (the deviation being from the probe molecule that is not bound to the target) and the concentration of the target in a sample.

**8.** An ESR analytical method according to any one of the preceding claims, wherein the paramagnetic moiety is bound to the probe molecule in a moiety of formula (II)

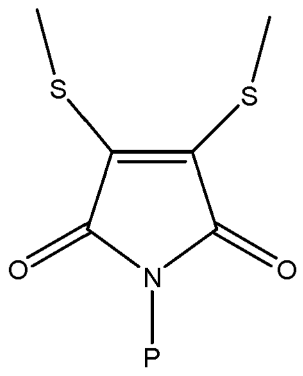

formula (II),

wherein each S forms part of a thioether linkage, such that the sulphur atom is bound to the probe molecule via a carbon atom, and P is the paramagnetic moiety.

**9.** An ESR analytical method according to claim 8, wherein the paramagnetic moiety P is of the formula (IV)

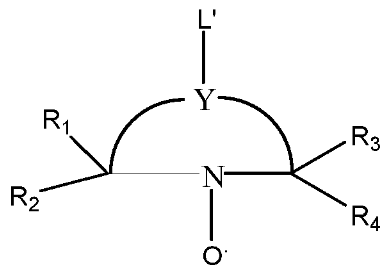

formula (IV)

wherein $R_1$ to $R_4$ are each independently an optionally substituted hydrocarbon, optionally containing from 1 to 4 carbon atoms, excluding any substituents;
Y is an optionally substituted hydrocarbon bridging group containing from 2 to 4 carbon atoms, and optionally one or more heteroatoms,
and L' is a linker species binding to N in formula (II), wherein L' is a single bond or $(CH_2)_n$, wherein n is from 0 to 4.

**10.** An ESR analytical method according to any one of the preceding claims, wherein a paramagnetic moiety is bound to the probe molecule in a moiety of formula (VIII)

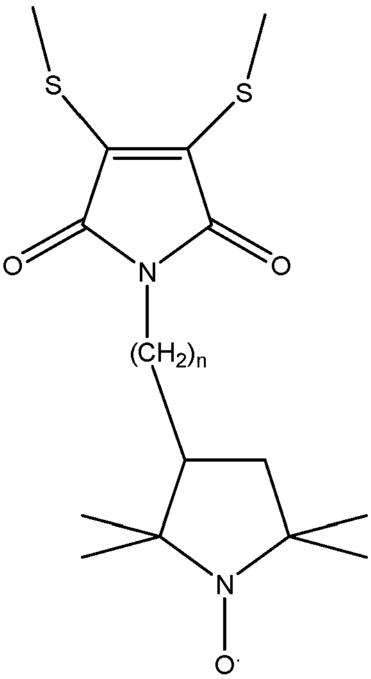

formula (VIII),

wherein each S forms part of a thioether linkage, such that the sulphur atom is bound to the probe molecule via a carbon atom and n is from 0 to 4.

**11.** A probe molecule capable of binding selectively to a target, wherein a paramagnetic moiety is bound to the probe molecule via two thioether linkages,

wherein each thioether linkage is a carbon-sulphur-carbon linkage and one carbon of the thioether linkage is part of the probe molecule, and the other carbon is part of either the paramagnetic moiety or a linker group that binds to the paramagnetic moiety
wherein the probe molecule is selected from an antibody fragment, an antibody and an aptamer.

**12.** A probe molecule according to claims 11, wherein the probe molecule is derived from a cystine stabilised single chain variable fragment of an antibody, wherein the disulphide bond of the cystine has been cleaved and each sulphur atom of each cysteine bonded to the paramagnetic moiety as part of a thioether linkage, optionally via a linker moiety.

## Patentansprüche

**1.** Analytisches Verfahren durch Elektronenspinresonanz (ESR), umfassend:

Bereitstellen eines Sondenmoleküls, das in der Lage ist, selektiv an ein Ziel zu binden, wobei eine paramagnetische Gruppierung über zwei Thioetherbindungen an das Sondenmolekül gebunden ist, wobei jede Thioetherbindung eine Kohlenstoff-Schwefel-Kohlenstoff-Bindung ist und ein Kohlenstoff der Thioetherbindung Teil des Sondenmoleküls ist und der andere Kohlenstoff entweder Teil der paramagnetischen Gruppierung oder einer Linker-Gruppe ist, die an die paramagnetische Gruppierung bindet;
Inkontaktbringen des Sondenmoleküls mit einer ersten Probe, die das Ziel umfassen kann oder nicht, um eine Mischprobe zu bilden, und Erlangen eines Elektronenspinresonanz-Signals der Mischprobe,
wobei das Sondenmolekül ausgewählt ist aus einem Antikörperfragment, einem Antikörper und einem Aptamer.

**2.** ESR-Analyseverfahren gemäß Anspruch 1, wobei die paramagnetische Gruppierung über zwei Thioetherbindungen an das Sondenmolekül gebunden ist, wobei das Schwefelatom in jeder Thioetherbindung Teil eines Cysteinrestes in dem Sondenmolekül ist.

3. ESR-Analyseverfahren gemäß Anspruch 1 oder Anspruch 2, wobei der Antikörper ein variables Einzelkettenfragment ist.

4. ESR-Analyseverfahren gemäß einem der vorherigen Ansprüche, wobei das Sondenmolekül von einem Cystin-stabilisierten einkettigen variablen Fragment eines Antikörpers abgeleitet ist, wobei die Disulfidbindung des Cystins gespalten wurde und jedes Schwefelatom von jedem Cystein als Teil einer Thioetherbindung, optional über eine Linker-Gruppierung, an die paramagnetische Gruppierung gebunden ist.

5. ESR-Analyseverfahren gemäß einem der vorherigen Ansprüche, wobei das Elektronenspinresonanz-Signal der Mischprobe mit einem Elektronenspinresonanz-Signal des Sondenmoleküls, das nicht an das Ziel gebunden ist, verglichen wird, wobei ein Unterschied im Signal das Vorhandensein des Ziels angibt.

6. ESR-Analyseverfahren gemäß einem der vorherigen Ansprüche, wobei die Mischprobe das Ziel enthält und die Konzentration des Ziels in der Mischprobe und/oder der ersten Probe aus dem Elektronenspinresonanz-Signal der Mischprobe bestimmt wird.

7. ESR-Analyseverfahren gemäß Anspruch 6, wobei die Konzentration unter Verwendung einer vorbestimmten Beziehung zwischen der mittleren quadratischen Abweichung des Elektronenspinresonanz-Signals des an das Ziel gebundenen Sondenmoleküls (wobei die Abweichung von dem nicht an das Ziel gebundenen Sondenmolekül ist) und der Konzentration des Ziels in einer Probe bestimmt wird.

8. ESR-Analyseverfahren gemäß einem der vorherigen Ansprüche, wobei die paramagnetische Gruppierung in einer Gruppierung von Formel (II)

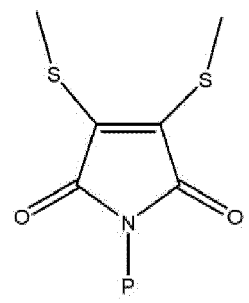

Formel (II)

an das Sondenmolekül gebunden ist,
wobei jedes S Teil einer Thioetherbindung ist, sodass das Schwefelatom über ein Kohlenstoffatom an das Sondenmolekül gebunden ist, und P die paramagnetische Gruppierung ist.

9. ESR-Analyseverfahren gemäß Anspruch 8, wobei der paramagnetische Gruppierung P von Formel (IV) ist,

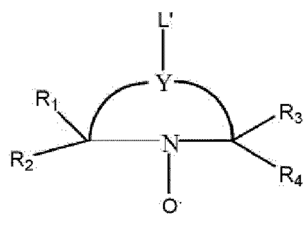

Formel (IV)

wobei $R_1$ bis $R_4$ jeweils unabhängig ein optional substituierter Kohlenwasserstoff sind, der optional 1 bis 4 Kohlenstoffatome enthält, wobei jegliche Substituenten ausgeschlossen sind;
Y eine optional substituierte Kohlenwasserstoff-Brückengruppe ist, die 2 bis 4 Kohlenstoffatome und optional ein oder mehrere Heteroatome enthält,
und L’ eine Linker-Spezies ist, die an N in Formel (II) bindet, wobei L’ eine Einfachbindung oder $(CH_2)_n$ ist, wobei n von 0 bis 4 ist.

10. ESR-Analyseverfahren gemäß einem der vorherigen Ansprüche, wobei eine paramagnetische Gruppierung in einer

Gruppierung von Formel (VIII) ist,

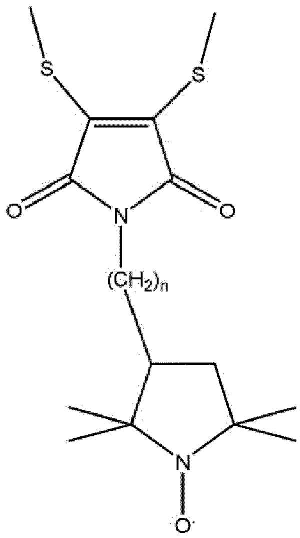

Formel (VIII)

**11.** Sondenmolekül, das in der Lage ist, selektiv an ein Ziel zu binden, wobei eine paramagnetische Gruppierung über zwei Thioetherbindungen an das Sondenmolekül gebunden ist,

wobei jede Thioetherbindung eine Kohlenstoff-Schwefel-Kohlenstoff-Bindung ist und ein Kohlenstoff der Thioetherbindung Teil des Sondenmoleküls ist und der andere Kohlenstoff entweder Teil der paramagnetischen Gruppierung oder einer Linker-Gruppe ist, die an die paramagnetische Gruppierung bindet,
wobei das Sondenmolekül ausgewählt ist aus einem Antikörperfragment, einem Antikörper und einem Aptamer.

**12.** Sondenmolekül gemäß Anspruch 11, wobei das Sondenmolekül von einem Cystin-stabilisierten einkettigen variablen Fragment eines Antikörpers abgeleitet ist, wobei die Disulfidbindung des Cystins gespalten wurde und jedes Schwefelatom von jedem Cystein als Teil einer Thioetherbindung, optional über eine Linker-Gruppierung, an die paramagnetische Gruppierung gebunden ist.

## Revendications

**1.** Un procédé analytique de résonance paramagnétique électronique (RPE) comprenant :

la fourniture d'une molécule sonde capable de se lier sélectivement à une cible, dans laquelle une fraction paramagnétique est liée à la molécule sonde via deux liaisons thioéther, dans laquelle chaque liaison thioéther est une liaison carbone-soufre-carbone et un carbone de la liaison thioéther fait partie de la molécule sonde, et l'autre carbone fait partie soit de la fraction paramagnétique, soit d'un groupe de liaison qui se lie à la fraction paramagnétique ;
la mise en contact de la molécule sonde avec un premier échantillon, qui peut comprendre ou non la cible, pour former un échantillon mélangé et
l'obtention d'un signal de résonance paramagnétique électronique de l'échantillon mélangé,
dans lequel la molécule sonde est choisie parmi un fragment d'anticorps, un anticorps et un aptamère.

**2.** Un procédé analytique de RPE selon la revendication 1, dans lequel la fraction paramagnétique est liée à la molécule sonde via deux liaisons thioéther, dans lequel l'atome de soufre dans chaque liaison thioéther fait partie d'un résidu de cystéine dans la molécule sonde.

**3.** Un procédé analytique de RPE selon la revendication 1 ou la revendication 2, dans lequel l'anticorps est un fragment variable à chaîne unique.

**4.** Un procédé analytique de RPE selon l'une quelconque des revendications précédentes, dans lequel la molécule sonde est dérivée d'un fragment variable à chaîne unique stabilisé par une cystine d'un anticorps, dans lequel la

liaison disulfure de la cystine a été clivée et chaque atome de soufre de chaque cystine est lié à la fraction paramagnétique en tant que partie d'une liaison thioéther, éventuellement via une fraction de liaison.

5. Un procédé analytique de RPE selon l'une quelconque des revendications précédentes, dans lequel le signal de résonance paramagnétique électronique de l'échantillon mélangé est comparé à un signal de résonance paramagnétique électronique de la molécule sonde qui n'est pas liée à la cible, une différence dans le signal indiquant la présence de la cible.

6. Un procédé analytique de RPE selon l'une quelconque des revendications précédentes, dans lequel l'échantillon mélangé contient la cible et la concentration de la cible dans l'échantillon mélangé et/ou dans le premier échantillon est déterminée à partir du signal de résonance paramagnétique électronique de l'échantillon mélangé.

7. Un procédé analytique de RPE selon la revendication 6, dans lequel la concentration est déterminée en utilisant une relation prédéterminée entre l'écart quadratique moyen du signal de résonance paramagnétique électronique de la molécule sonde liée à la cible (l'écart étant par rapport à la molécule sonde qui n'est pas liée à la cible) et la concentration de la cible dans un échantillon.

8. Un procédé analytique de RPE selon l'une quelconque des revendications précédentes, dans lequel la fraction paramagnétique est liée à la molécule sonde dans une fraction de formule (II)

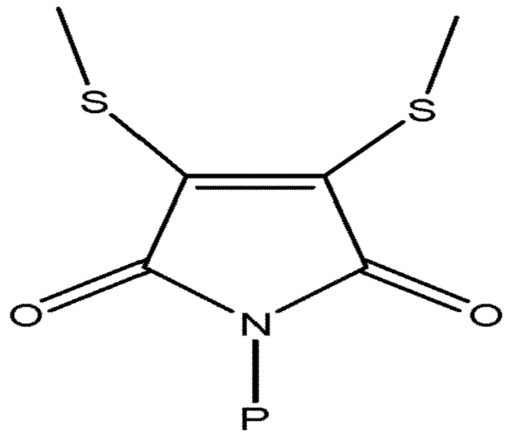

formule (II),

dans laquelle chaque S fait partie d'une liaison thioéther, de sorte que l'atome de soufre est lié à la molécule sonde via un atome de carbone, et P est la fraction paramagnétique.

9. Un procédé analytique de RPE selon la revendication 8, dans lequel la fraction paramagnétique P est de la formule (IV)

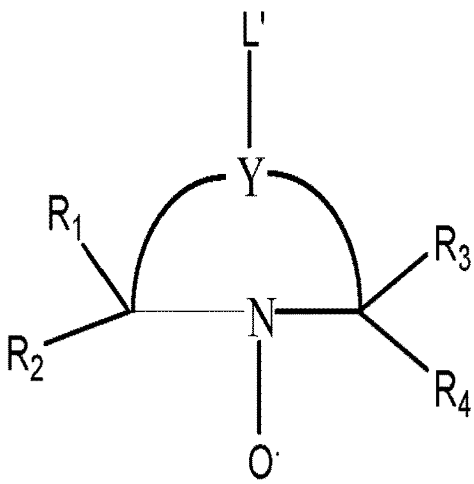

formule (IV),

dans laquelle $R_1$ à $R_4$ sont chacun indépendamment un hydrocarbure éventuellement substitué, contenant éventuellement de 1 à 4 atomes de carbone, à l'exclusion de tout substituant ;
Y est un groupe de pontage d'hydrocarbure éventuellement substitué, contenant de 2 à 4 atomes de carbone, et éventuellement un ou plusieurs hétéroatomes,
et L' est une espèce chimique de liant se liant à N dans la formule (II), dans laquelle L' est une liaison simple ou $(CH_2)_n$, dans laquelle n est compris entre 0 et 4.

**10.** Un procédé analytique de RPE selon l'une quelconque des revendications précédentes, dans lequel une fraction paramagnétique est liée à la molécule sonde dans une fraction de formule (VIII)

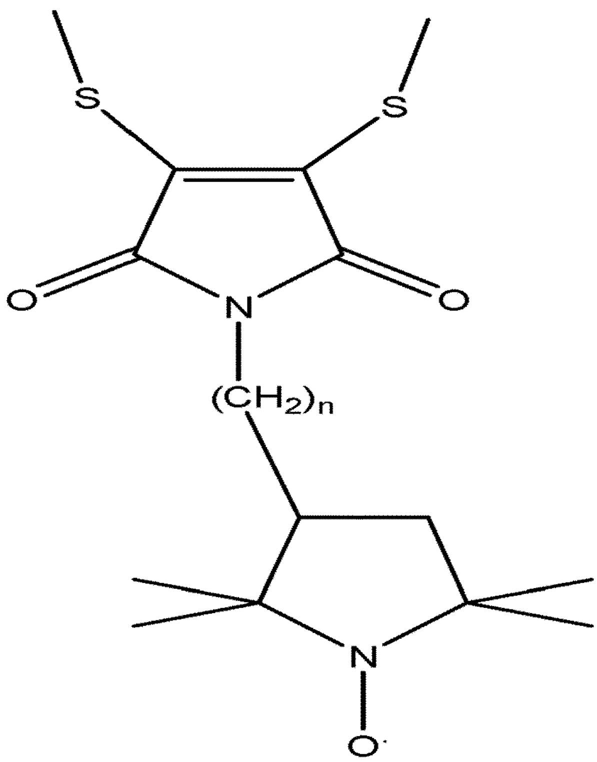

formule (VIII),

dans laquelle chaque S fait partie d'une liaison thioéther, de sorte que l'atome de soufre est lié à la molécule sonde

via un atome de carbone et n est compris entre 0 et 4.

**11.** Une molécule sonde capable de se lier sélectivement à une cible, dans laquelle une fraction paramagnétique est liée à la molécule sonde via deux liaisons thioéther,

dans laquelle chaque liaison thioéther est une liaison carbone-soufre-carbone et un carbone de la liaison thioéther fait partie de la molécule sonde, et l'autre carbone fait partie soit de la fraction paramagnétique, soit d'un groupe de liaison qui se lie à la fraction paramagnétique
dans laquelle la molécule sonde est choisie parmi un fragment d'anticorps, un anticorps et un aptamère.

**12.** Une molécule sonde selon la revendication 11, dans laquelle la molécule sonde est dérivée d'un fragment variable à chaîne unique stabilisé par une cystine d'un anticorps, dans laquelle la liaison disulfure de la cystine a été clivée et chaque atome de soufre de chaque cystine est lié à la fraction paramagnétique en tant que partie d'une liaison thioéther, éventuellement via une fraction de liaison.

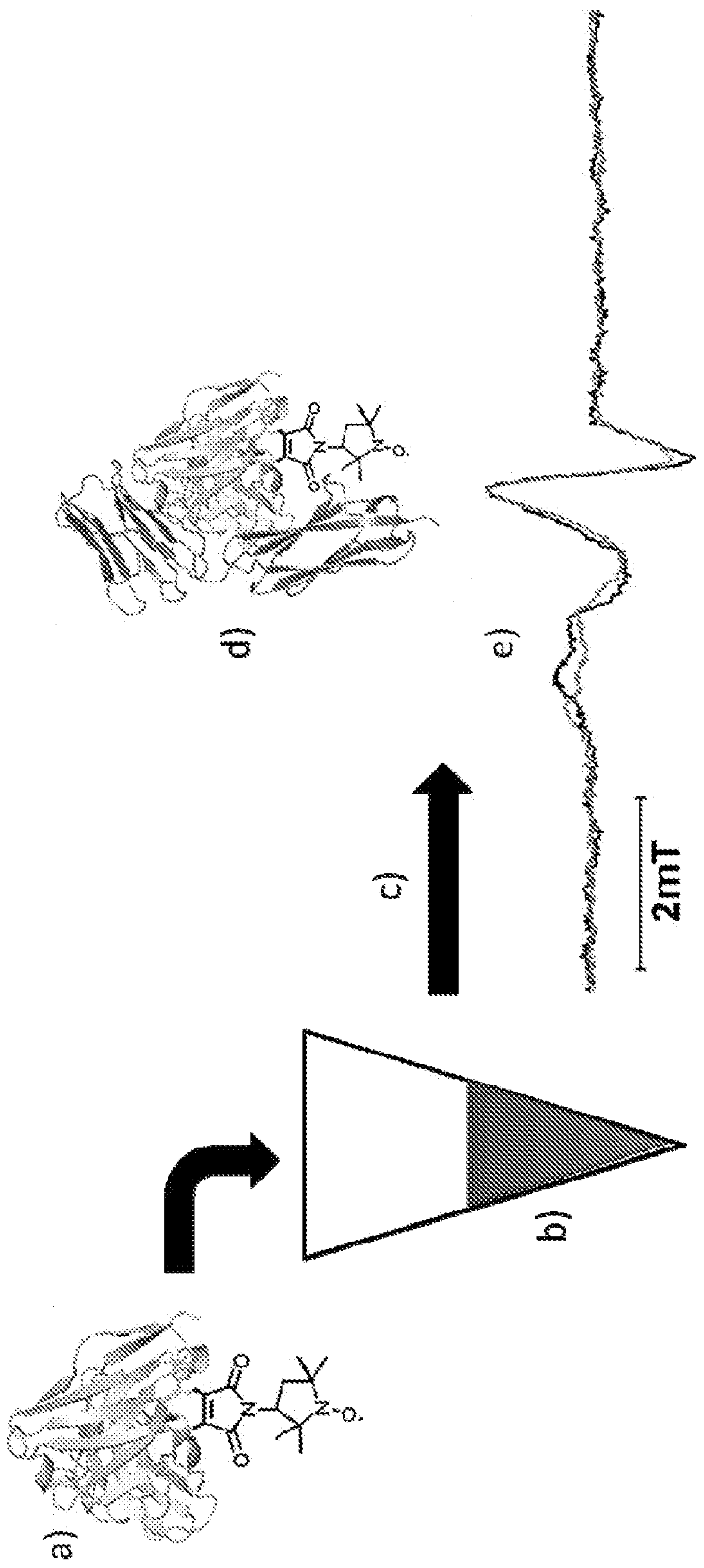


Fig. 1

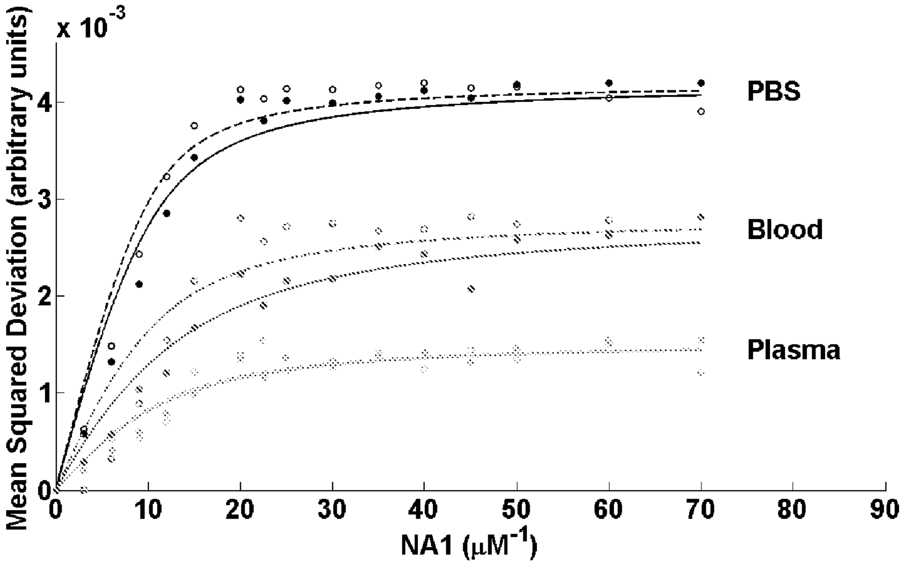


Fig. 2(a)

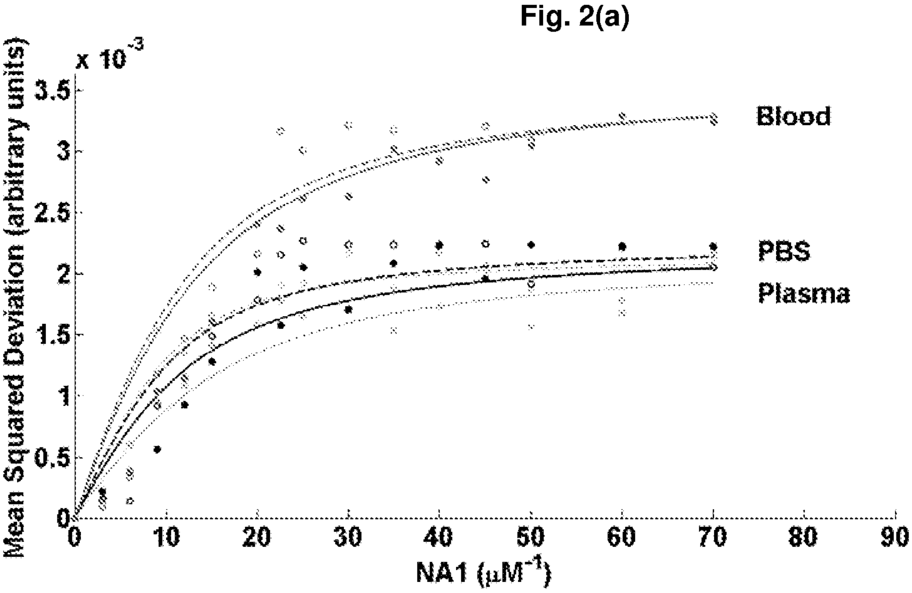


Fig. 2(b)

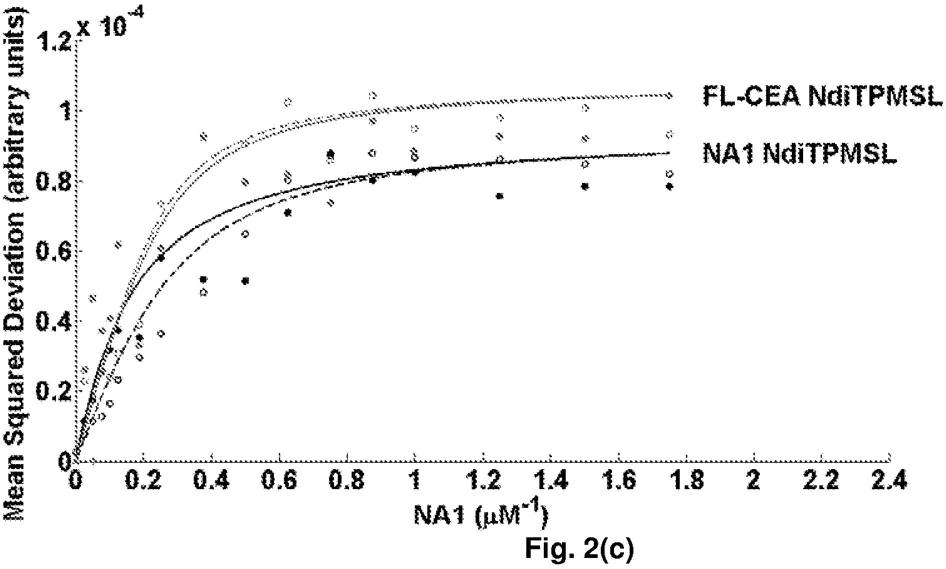


Fig. 2(c)

d)

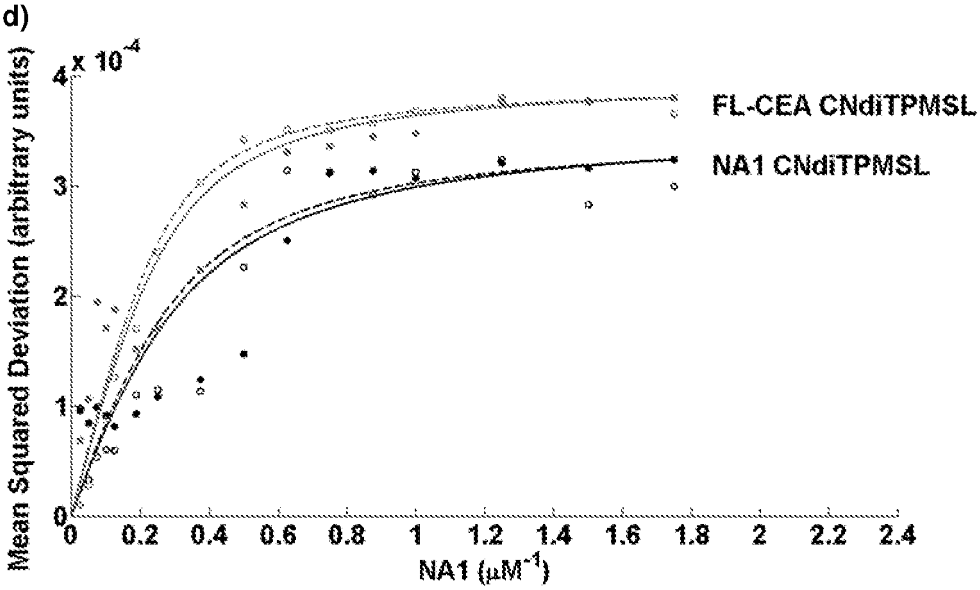

x 10^-4
4
3
2
1
0
Mean Squared Deviation (arbitrary units)
0
0.2
0.4
0.6
0.8
1
1.2
1.4
1.6
1.8
2
2.2
2.4
NA1 (μM^-1)
FL-CEA CNdiTPMSL
NA1 CNdiTPMSL


Fig. 2(d)

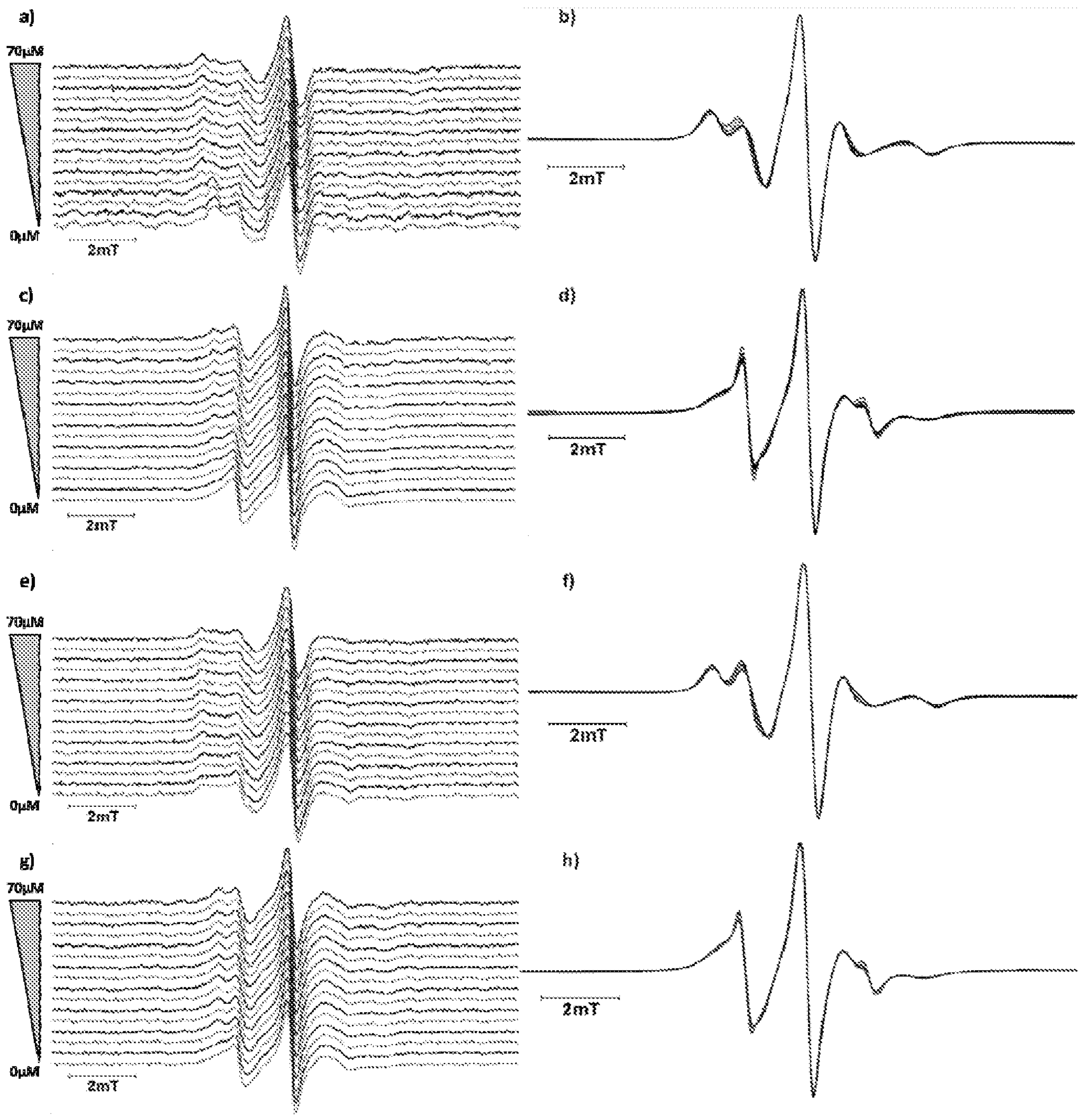
a)
70μM
0μM
2mT
b)
2mT
c)
70μM
0μM
2mT
d)
2mT
e)
70μM
0μM
2mT
f)
2mT
g)
70μM
0μM
2mT
h)
2mT

Figs 3(a) to 3(h)

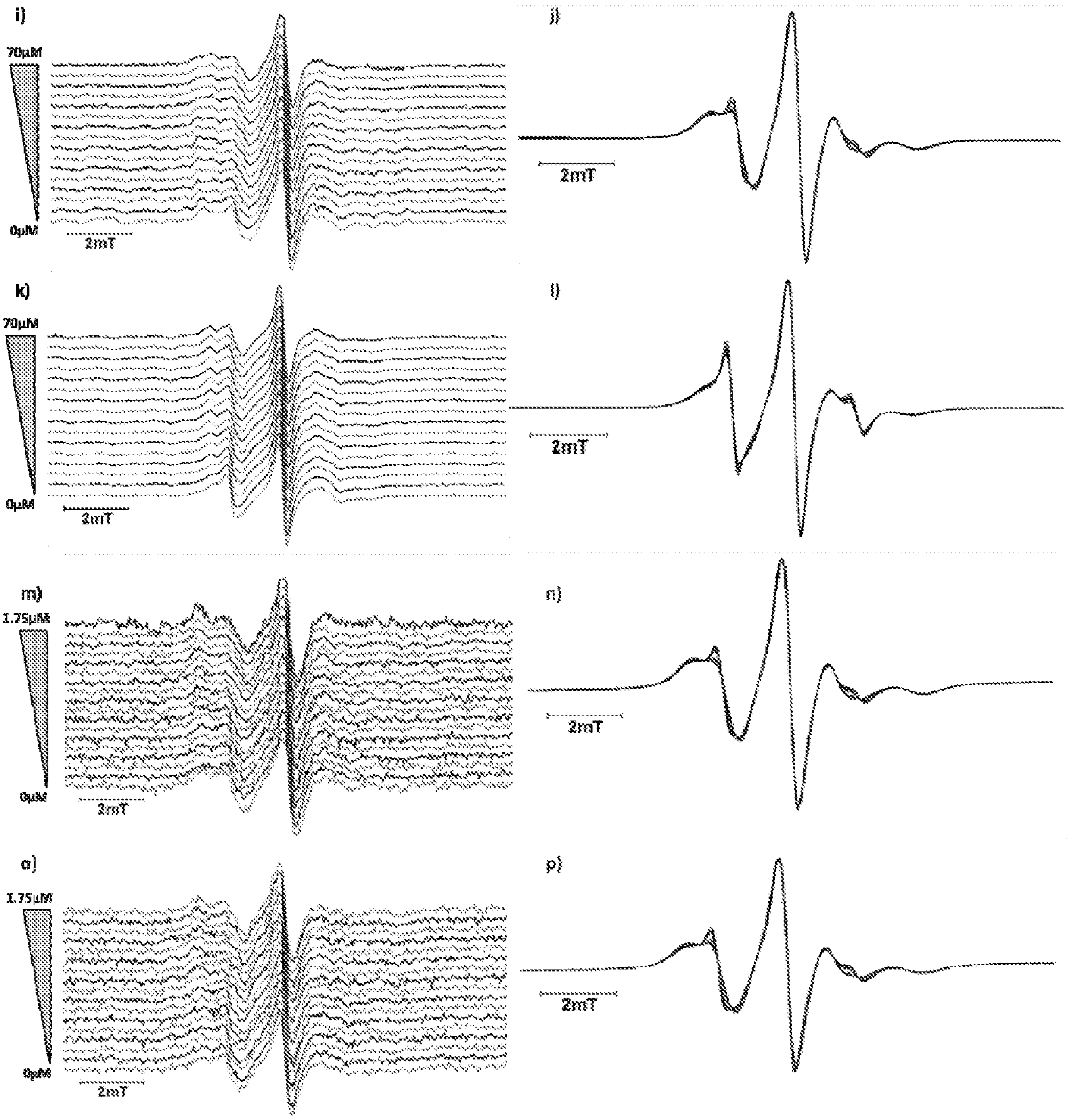
i)
70μM
0μM
2mT
j)
2mT
k)
70μM
0μM
2mT
l)
2mT
m)
1.75μM
0μM
2mT
n)
2mT
o)
1.75μM
0μM
2mT
p)
2mT

Figs. 3(i) to 3(p)

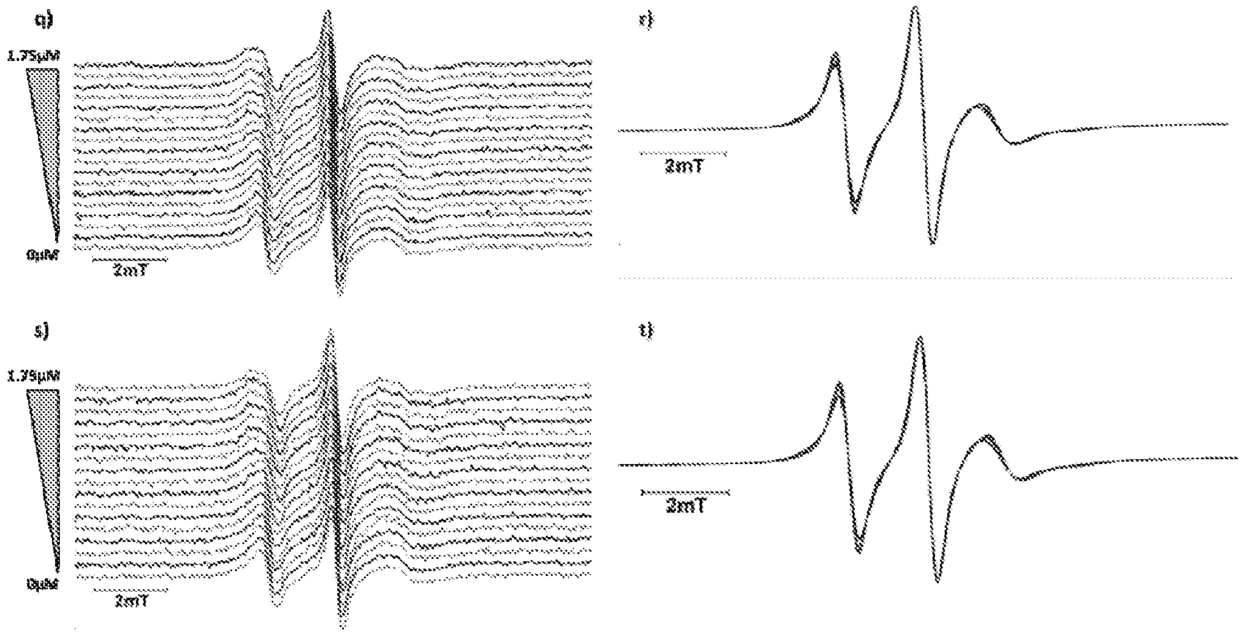


**Figs. 3(q) to 3(t)**

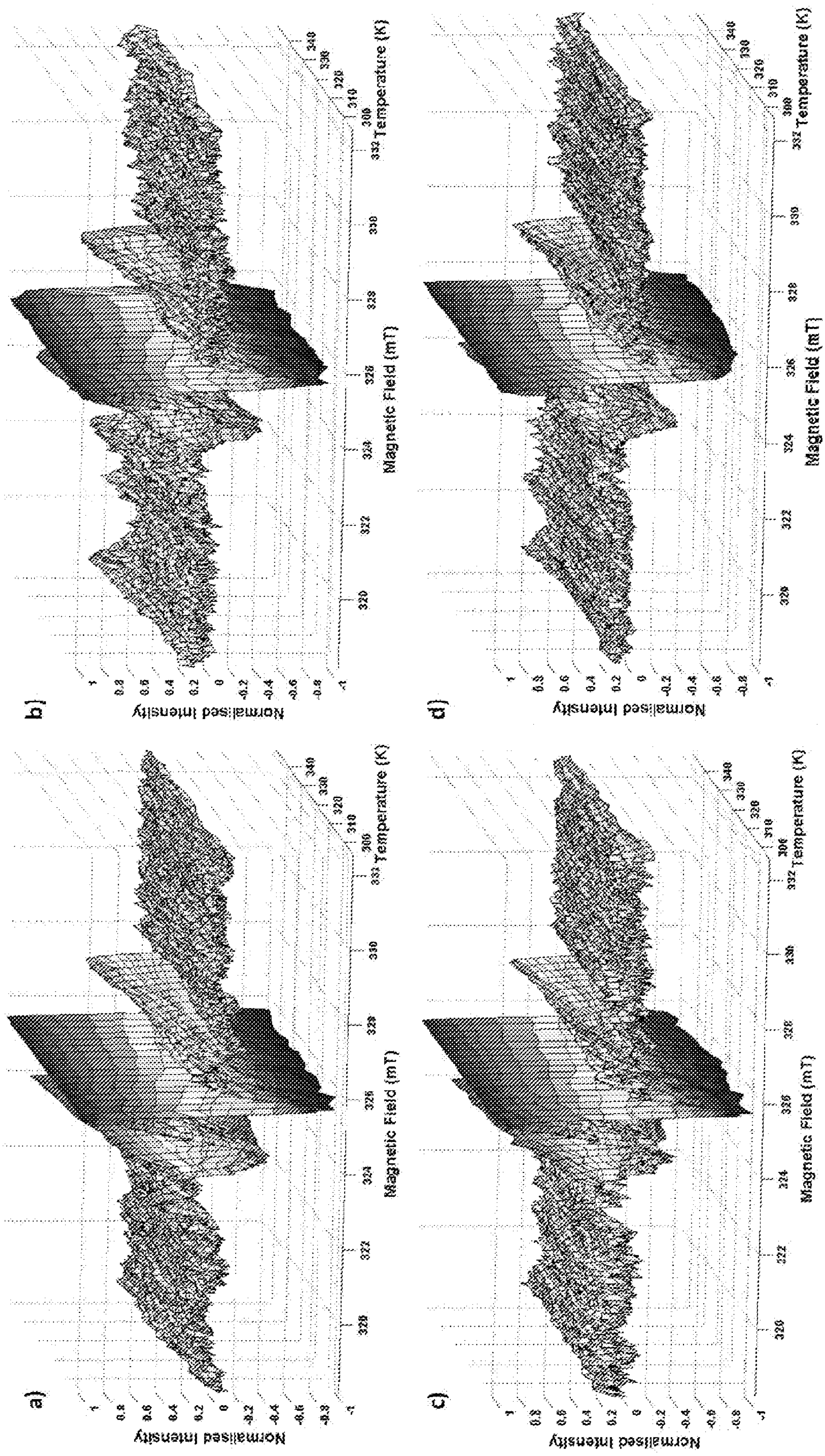
a)
b)
c)
d)
Normalised Intensity
Magnetic Field (mT)
Temperature (K)

Figs. 4(a) to 4(d)

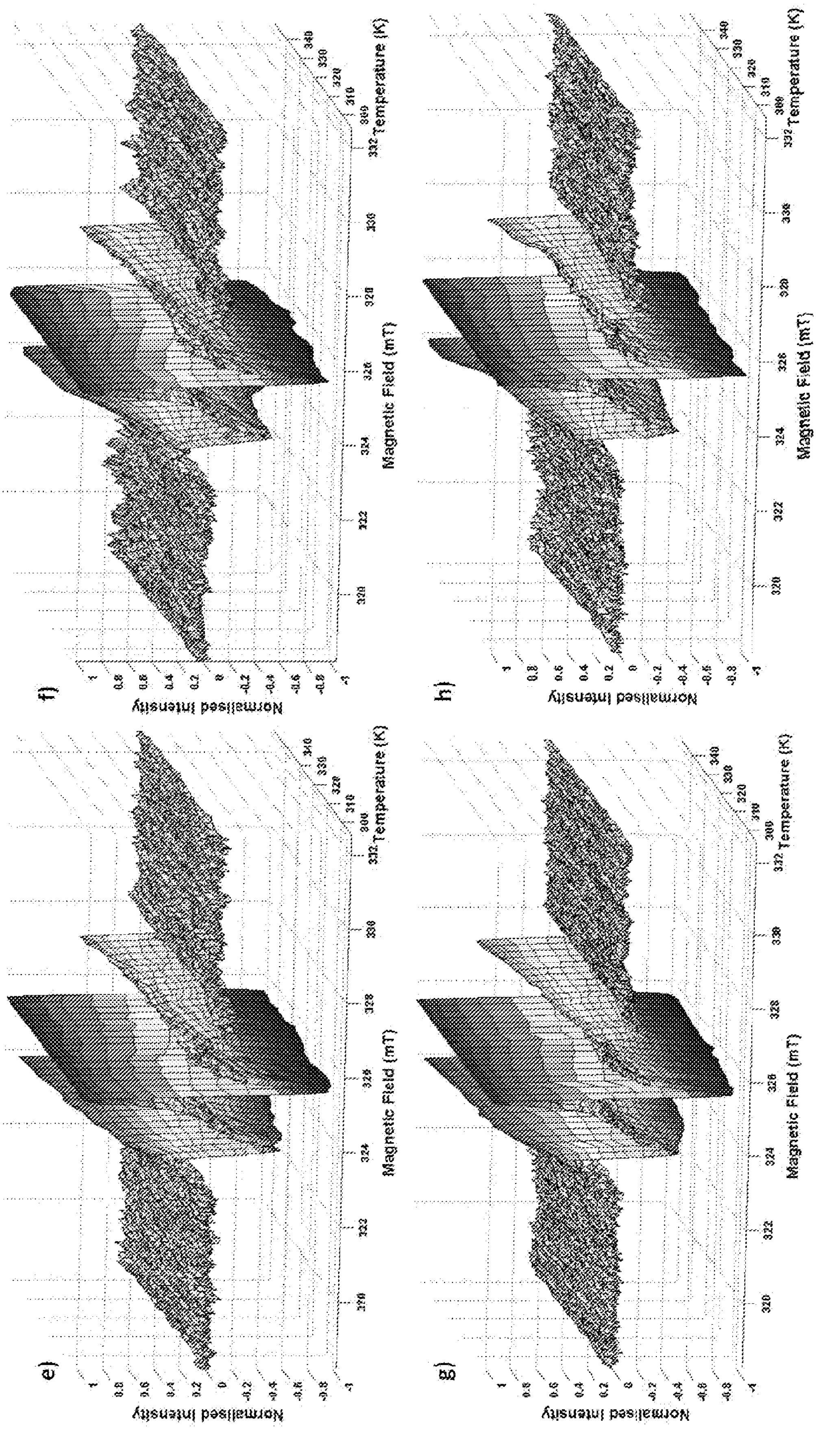
e)
f)
g)
h)
Normalised Intensity
Magnetic Field (mT)
Temperature (K)

Figs. 4(e) to 4(h)

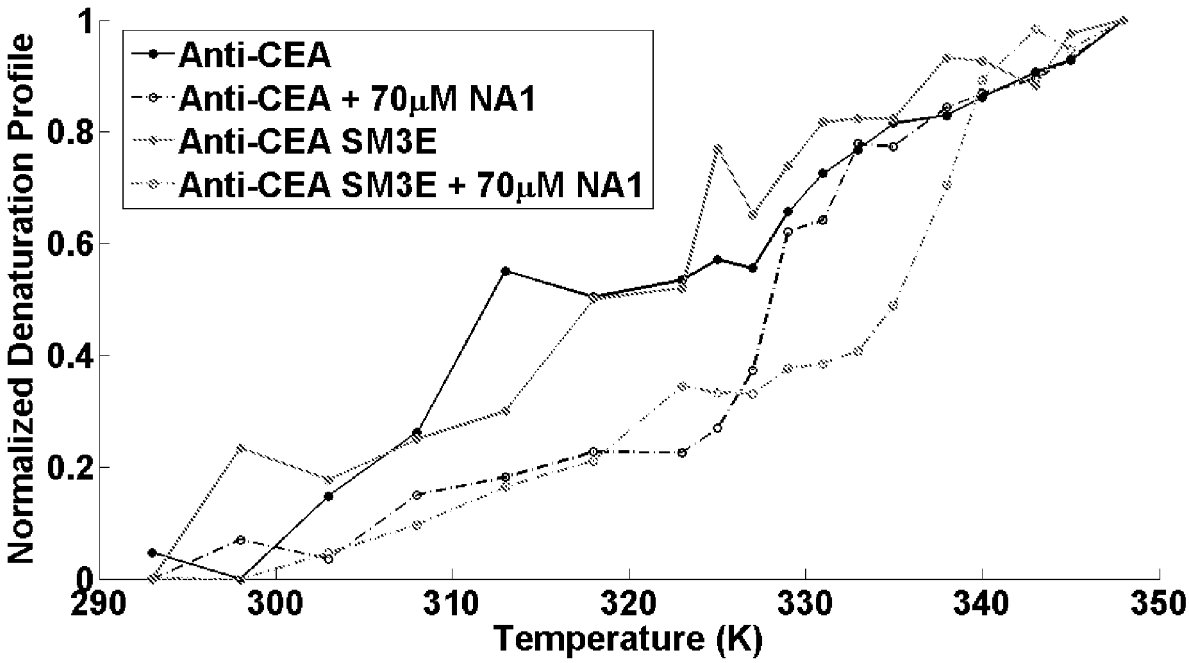
Anti-CEA
Anti-CEA + 70μM NA1
Anti-CEA SM3E
Anti-CEA SM3E + 70μM NA1
Normalized Denaturation Profile
Temperature (K)
0
0.2
0.4
0.6
0.8
1
290
300
310
320
330
340
350

Fig. 5(a)

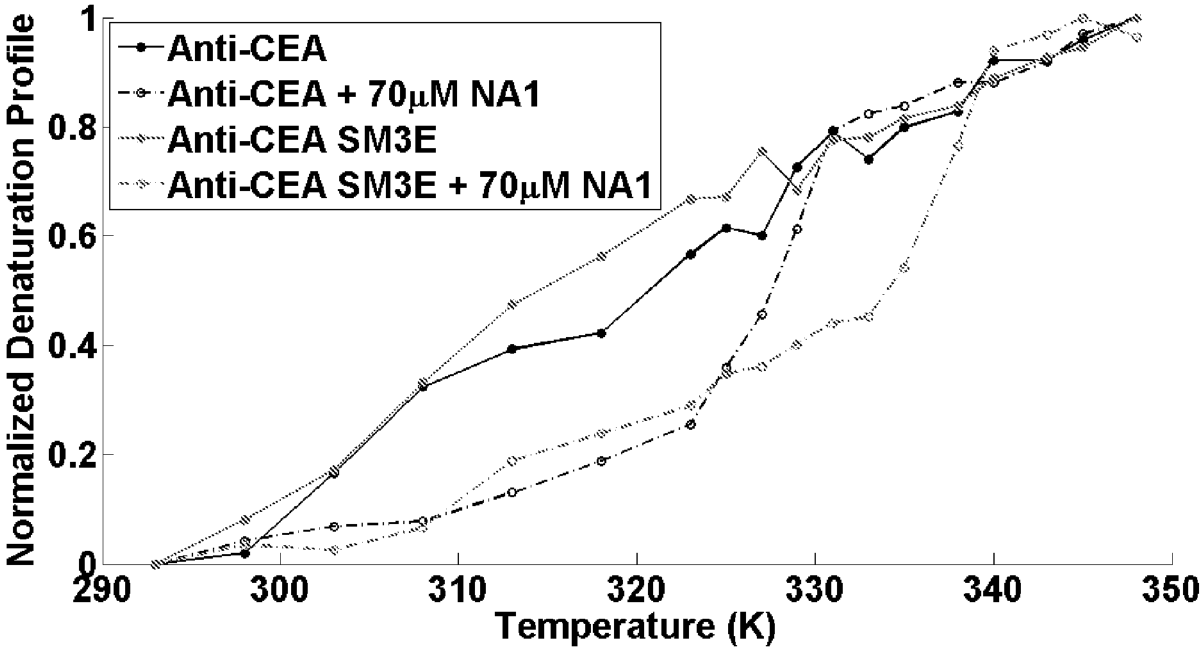
Anti-CEA
Anti-CEA + 70μM NA1
Anti-CEA SM3E
Anti-CEA SM3E + 70μM NA1
Normalized Denaturation Profile
Temperature (K)
0
0.2
0.4
0.6
0.8
1
290
300
310
320
330
340
350

Fig 5(b)

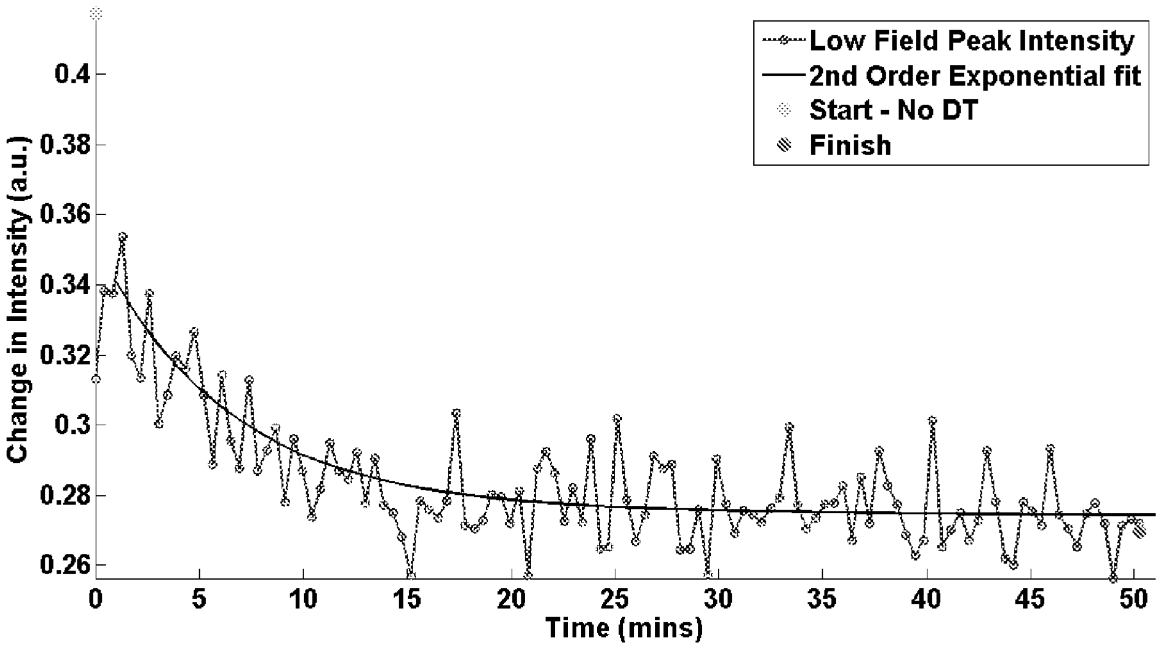
Low Field Peak Intensity
2nd Order Exponential fit
Start - No DT
Finish
Change in Intensity (a.u.)
Time (mins)
0.4
0.38
0.36
0.34
0.32
0.3
0.28
0.26
0
5
10
15
20
25
30
35
40
45
50

Fig. 6(a)

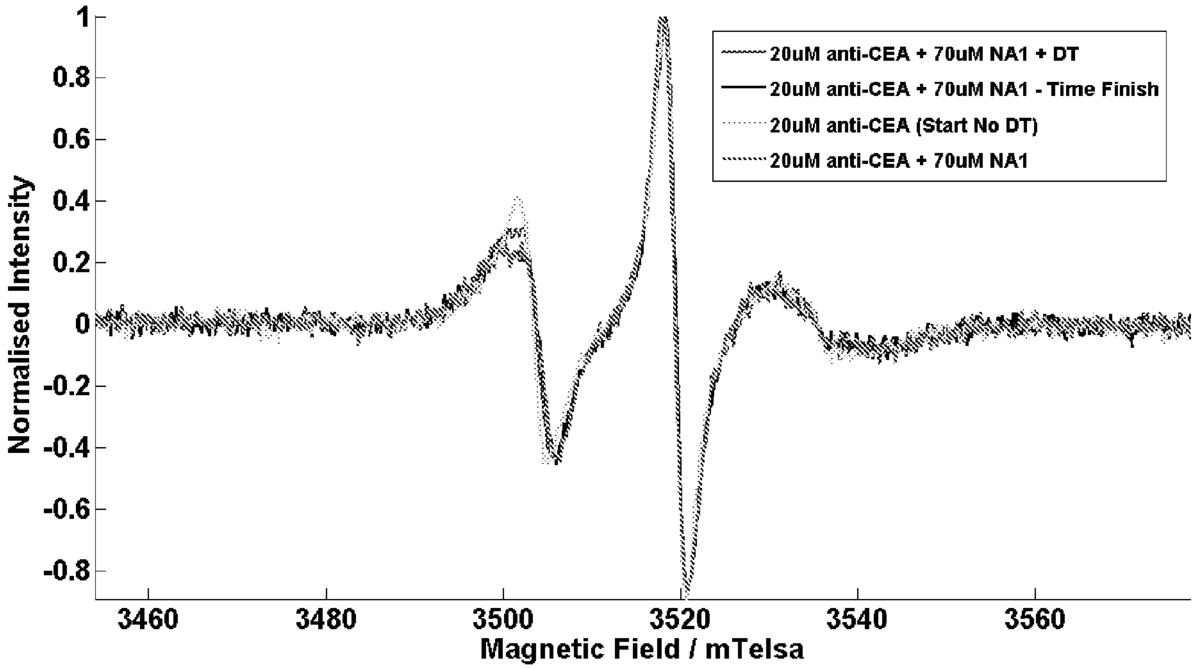
20uM anti-CEA + 70uM NA1 + DT
20uM anti-CEA + 70uM NA1 - Time Finish
20uM anti-CEA (Start No DT)
20uM anti-CEA + 70uM NA1
Normalised Intensity
Magnetic Field / mTelsa
1
0.8
0.6
0.4
0.2
0
-0.2
-0.4
-0.6
-0.8
3460
3480
3500
3520
3540
3560

Fig. 6(b)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3690834 A
- WO 3690834 A, Golstein, A. Leute R. K. Ullman E. F.


### Non-patent literature cited in the description


- *Nat Biotechnol.,* October 1996, vol. 14 (10), 1239-45
- **MIZUNO,T.M.K.T.Y.O.M.T.T.** *Metal-ion-dependent GFP emission in vivo by combining a circularly permutated green fluorescent protein with an engineered metal-ionbinding coiled-coil.,* vol. 129 (20), 11383
- **KATZ,E.S.-H.-I.L.W.** *Electrical contacting of glucose oxidase in a redox-active rotaxane configuration,* vol. 43 (20), 3300
- **YAO,C.Z.T.T.J.W.R.C.Q.C.M.Z.B.H.J.F.W.** *Hybridization assay of hepatitis B virus by QCM peptide nucleic acid biosensor,* vol. 23 (20), 885
- **KAWAZUMI,H.** *Compact surface plasmon resonance (SPR) immunosensor using multichannel for simultaneous detection of small molecule compounds,* vol. 108 (20), 796
- **JESCHKE,G. ; POLYHACH,Y.** Distance measurements on spin-labelled biomacromolecules by pulsed electron paramagnetic resonance. *Phys. Chem. Chem. Phys.,* 2007, vol. 9, 1895-1910
- **KLARE,J.P. ; STEINHOFF,H.J.** Spin labeling EPR. *Photosynth. Res.,* 2009, vol. 102, 377-390
- **SOWA,G.** *Site-directed spin labeling studies on nucleic acid structure and dynamics,* vol. 82 (20), 197
- **CZOGALLA,A.P.A.J.A.S.A.** *Attaching a spin to a protein -- site-directed spin labeling in structural biology,* vol. 54 (20), 244
- **CZOGALLA,A. ; PIECIUL,A. ; JEZIERSKI,A. ; SIKORSKI,A.F.** Attaching a spin to a protein -- site-directed spin labeling in structural biology. *Acta Biochim. Pol.,* 2007, vol. 54, 235-244
- **JAKOBSEN,U. ; SHELKE,S.A. ; VOGEL,S. ; SIGURDSSON,S.T.** Site-directed spin-labeling of nucleic acids by click chemistry: detection of abasic sites in duplex DNA by EPR spectroscopy. *J. Am. Chem. Soc.,* 2010, vol. 132, 10424-10428
- **LANDSBERGER,F.R. ; LENARD,J ; PAXTON,J. ; COMPANS,R.W.** Spin-labeled electron spin resonance study of the lipid-containing membrane of influenza virus. *Proc. Natl. Acad. Sci. U. S. A,* 1971, vol. 68, 2579-2583
- **COLUMBUS,L. ; HUBBELL,W.L.** A new spin on protein dynamics. *Trends Biochem. Sci.,* 2002, vol. 27, 288-295
- **OPPENHEIM,S.B.G.R.V.** *Relationship of rotational correlation time from EPR spectroscopy and protein-membrane interaction,* vol. 118 (19), 139
- **ULLMAN,E.F.** *Homogeneous immunoassays: Historical perspective and future promise,* vol. 76 (19), 788
- **KOHLER,J. ; DISSELHORST,J.A.J.M. ; DONCKERS,M.C.J.M. ; GROENEN,E.J.J. ; SCHMIDT,J. ; MOERNER,W.E.** Magnetic resonance of a single molecular spin. *Nature,* 1993, vol. 363, 242-244
- **HSIA,J.C.** *Spin-labeling as a general method in studying antibody active site,* vol. 129 (19
- **HSIA,J.C.** *Membrane immunoassay: principle and applications of spin membrane immunoassay,* vol. 308 (19), 48
- **TIMOFEEV,V.P.** *Selective spin-labelling of an N-acetylneuraminic acid residue in the Fab-region oligosaccharide of immunoglobulin M.,* vol. 17 (19), 176
- **WILLAN,K.J.** *Specific spin labelling of the Fc region of immunoglobulins,* vol. 80 (19), 136
- **REVERBERI,R.** *Factors affecting the antigen-antibody reaction,* vol. 5 (20), 40
- **NESMELOV,Y.E. ; SUREK,J.T. ; THOMAS,D.D.** Enhanced EPR Sensitivity from a Ferroelectric Cavity Insert. *Journal of Magnetic Resonance,* 2001, vol. 153, 7-14
- **STEFAN STOLL ; ARTHUR SCHWEIGER.** EasySpin, a comprehensive software package for spectral simulation and analysis in EPR. *J. Magn. Reson.,* 2006, vol. 178 (1), 42-55
- **TOLNER, B. ; SMITH, L. ; BEGENT, R.H. ; CHESTER, K.A.** Production of recombinant protein in Pichia pastoris by fermentation. *Nat Protoc,* 2006, vol. 1, 1006-1021
- **TOLNER, B. ; BHAYSAR, G. ; FOSTER, B. ; VIGOR, K. ; CHESTER, K.** Laboratory Protocols in Fungal Biology. Springer Science and Business Media, 2012

- **SAINZ-PASTOR, N. et al.** Deglycosylation to obtain stable and homogeneous Pichia pastoris-expressed N-A1 domains of carcinoembryonic antigen. *Int J Biol Macromol,* 2006, vol. 39, 141-150